(19) Europäisches Patentamt
European Patent Office
Office européen des brevets

(11) Veröffentlichungsnummer: **0 096 142**
**B1**

# (12) EUROPÄISCHE PATENTSCHRIFT

(45) Veröffentlichungstag der Patentschrift: **18.01.89**

(21) Anmeldenummer: **82810539.5**

(22) Anmeldetag: **13.12.82**

(51) Int. Cl.⁴: **C 07 D 207/48,** C 07 D 401/04, C 07 D 405/04, C 07 D 409/04, C 07 F 9/40, A 01 N 43/34, A 01 N 57/24 // C07D207/32, C07D207/34, C07D207/42

## (54) Mikrobizide Sulfenyl-Pyrrole.

(30) Priorität: **18.12.81 CH 8110/81**

(43) Veröffentlichungstag der Anmeldung: **21.12.83 Patentblatt 83/51**

(45) Bekanntmachung des Hinweises auf die Patenterteilung: **18.01.89 Patentblatt 89/3**

(84) Benannte Vertragsstaaten: **AT BE CH DE FR GB IT LI NL**

(56) Entgegenhaltungen:
**EP-A- 0 008 367**
**EP-A- 0 010 616**
**CA-A- 689 434**
**DE-A- 2 614 935**
**DE-A- 2 636 076**
**DE-A- 2 903 458**
**DE-A- 2 927 480**
**US-A- 2 888 462**
**US-A- 3 499 030**

**Chemie der Pflanzenschutz- und Schädlingsbekämpfungsmittel, Band 4, R. WEGLER, Springer-Verlag, Berlin, 1977, S. 170-195-, 200.**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel (CH)**

(72) Erfinder: **Nyfeler, Robert, Dr., Bärenfelserstrasse 8, CH-4057 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 096 142 B1

## Beschreibung

Die vorliegende Erfindung betrifft neue N-sulfenylierte Pyrrol-derivate, deren Herstellung, sowie mikrobizide Mittel, die als Wirkstoff mindestens eine der Titelverbindungen enthalten. Die Erfindung betrifft ferner die Herstellung der genannten Mittel und die Verwendung der neuen Wirkstoffe und Mittel zur Bekämpfung schädlicher Mikroorganismen, insbesondere pflanzenschädigender Pilze.

Es werden hierin neue Verbindungen der allgemeinen Formel I

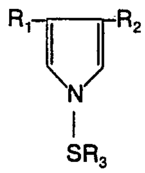
(I)

beschrieben, worin

$R_1$ für eines der Fragmente [X,Y,Z(Phenyl)], [X,Y,Z(Biphenyl)], [U,V,W(Pyridyl)], [U,V,W(Furyl)] oder [U,V,W(Thienyl)] steht, wobei X, Y und Z unabhängig voneinander für Wasserstoff, Halogen, $C_1$–$C_4$-Alkyl, $C_1$–$C_3$-Haloalkyl, Di($C_1$–$C_4$-alkyl)amino, Nitro, Cyano, –COO($C_1$–$C_4$-alkyl), –CON($C_1$–$C_4$-alkyl)$_2$ oder die Gruppe –E–$R_4$ bedeuten, wobei E für –O–, –S–, –SO– oder –$SO_2$– steht, $R_4$ unsubstituiertes $C_1$–$C_6$-Alkyl, unsubstituiertes oder durch Halogen substituiertes $C_3$–$C_5$-Alkenyl, unsubstituiertes oder durch Halogen oder Hydroxy substituiertes $C_3$–$C_5$-Alkinyl, [X,Y,Z(Phenyl)] oder –$CH_2$-[X,Y,Z(Phenyl)] bedeutet; U, V, W unabhängig voneinander für Wasserstoff, Halogen oder $C_1$–$C_4$-Alkyl stehen;

$R_2$ für –COO($C_1$–$C_6$-alkyl), –CO–($C_1$–$C_6$-alkyl), –CO–N($C_1$–$C_6$-alkyl)$_2$, Cyano, Nitro, –$SO_2$–($C_1$–$C_6$-alkyl), –P(O)–($C_1$–$C_6$-alkoxy)$_2$, –SO–($C_1$–$C_6$-alkyl) oder für –$SO_2$–N($C_1$–$C_6$-alkyl)$_2$ steht; und

$R_3$ $C_1$–$C_3$-Haloalkyl bedeutet.

Die Symbole [X,Y,Z(Phenyl)], [X,Y,Z(Biphenyl)], [U,V,W(Pyridyl)], [U,V,W(Furyl)] und [U,V,W(Trienyl)] stehen hier und im folgenden jeweils für folgende Gruppierung:

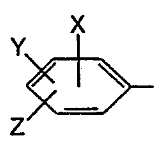
,
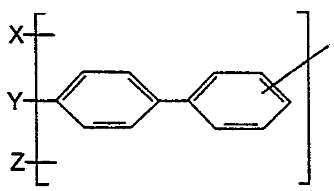
,
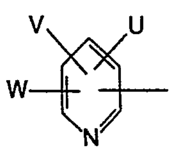
,
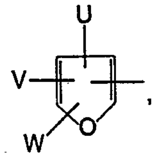
,
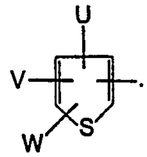
.

Unter dem Begriff Alkyl selbst oder als Bestandteil eines anderen Substituenten sind je nach Zahl der angegebenen Kohlenstoffatome beispielsweise folgende Gruppen zu verstehen: Methyl, Ethyl, Propyl, Butyl, Pentyl, Hexyl usw., sowie ihre Isomeren, wie z.B. Isopropyl, Isobutyl, tert.-Butyl, Isopentyl usw. Haloalkyl steht für einen einfach bis perhalogenierten Alkylsubstituenten, wie z.B. $CH_2Cl$, $CHCl_2$, $CCl_3$, $CH_2Br$, $CHBr_2$, $CBr_3$, $CH_2F$, $CHF_2$, $CF_3$, $CCl_2F$, $CCl_2$–$CHCl_2$, $CH_2CH_2F$, $CJ_3$ usw. Unter Halogen soll hier und im folgenden Fluor, Chlor, Brom oder Jod, vorzugsweise Fluor, Chlor oder Brom, verstanden werden. $C_3$–$C_5$-Alkenyl steht für einen ungesättigten, aliphatischen Rest mit einer oder mit mehreren Doppelbindungen und besteht aus maximal 5 Kohlenstoffatomen, so z.B. für Propenyl-(1), Allyl, Butenyl-(1), Butenyl-(2), Butenyl-(3), $CH_3CH{=}CHCH{=}CH$– usw. Der Ausdruck Pyridyl steht für 2-, 3- und 4-Pyridyl, Furyl für 2- und 3-Furyl, Thienyl für 2- und 3-Thienyl, Biphenyl für Biphenyl-4-yl, Biphenyl-3-yl und Biphenyl-2-yl, Alkinyl steht insbesondere für Propargyl.

Die Verbindungen der Formel I sind unter Normalbedingungen stabile Öle, Harze oder überwiegend kristalline Feststoffe, die sich durch äusserst wertvolle mikrobizide Eigenschaften auszeichnen. Sie lassen sich beispielsweise auf dem Agrarsektor oder verwandten Gebieten präventiv und kurativ zur Bekämpfung pflanzenschädigender Mikroorganismen einsetzen. Die erfindungsgemässen Wirkstoffe der Formel I zeichnen sich in weiten Anwendungskonzentrationen durch eine hohe fungizide Aktivität und problemlose Anwendung aus.

Aufgrund ihrer ausgeprägten mikrobiziden Wirkung sind diejenigen Wirksubstanzen der Formel I bevorzugt, die folgende Substituententypen oder Kombinationen dieser Substituententypen untereinander aufweisen:

Bei $R_1$:

a) [X,Y,Z(Phenyl)], [X,Y,Z(Biphenyl-4-yl)], [X,Y,Z(Biphenyl-3-yl)], [X,Y,Z(Biphenyl-2-yl)], [U,V,W(2-Pyridyl)], [U,V,W(3-Pyridyl)], [U,V,W(4-Pyridyl)], [U,V,W(2-Furyl)], [U,V,W(3-Furyl)], [U,V,W(2-Thienyl)], [U,V,W(3-Thienyl)];
b) [X,Y,Z(Phenyl)], [X,Y,Z(Biphenyl-4-yl)], [U,V,W(2-Pyridyl)], [U,V,W(3-Pyridyl)], [U,V,W(2-Furyl)], [U,V,W(2-Thienyl)];
c) [X,Y,Z(Phenyl)], [U,V,W(2-Furyl)];
d) [X,Y,Z(Phenyl)].

Bei X,Y,Z:

a) H, Halogen, $C_1$–$C_3$-Alkyl, $C_1$–$C_3$-Haloalkyl, Di($C_1$–$C_3$-alkyl)amino, Nitro, Cyano, –COO($C_1$–$C_3$-alkyl), –CON($C_1$–$C_3$-alkyl)$_2$, $C_1$–$C_4$-Alkoxy, $C_1$–$C_4$-Thioalkyl, $C_3$–$C_5$-Alkenyloxy, $C_3$–$C_5$-Haloalkenyloxy, Phenoxy, Benzyloxy;
b) H, F, Cl, Br, $CH_3$, $C_2H_5$, $CF_3$, –N($CH_3$)$_2$, –N($C_2H_5$)$_2$, –$COOCH_3$, –$COOC_2H_5$, –CON($CH_3$)$_2$, $C_1$–$C_4$-Alkoxy, $C_1$–$C_4$-Thioalkyl, $C_3$–$C_5$-Alkenyloxy, $C_3$–$C_5$-Haloalkenyloxy, Phenoxy, Benzyloxy;
c) H, Cl, Br, $CH_3$, $C_2H_5$, $CF_3$, –N($CH_3$)$_2$, –$COOCH_3$, –CON($CH_3$)$_2$, $C_1$–$C_4$-Alkoxy, $C_1$–$C_4$-Alkylthio, $C_3$-Alkenyloxy, Phenoxy, Benzyloxy;
d) H, Cl, Br, $CH_3$, $C_1$–$C_4$-Alkoxy, $C_1$–$C_4$-Alkylthio, $C_3$-Alkenyloxy.

Bei U,V,W:

a) H, Halogen, $C_1$–$C_4$-Alkyl;

b) H, Cl, Br, $C_1$–$C_2$-Alkyl;
c) H, Cl, Br, $CH_3$.

Bei $R_2$:
a) –COO($C_1$–$C_3$-alkyl), –CO–($C_1$–$C_3$-alkyl), –CO–N($C_1$–$C_3$l-alkyl), Cyano, Nitro, –$SO_2$–($C_1$–$C_3$-alkyl), –P(O)($C_1$–$C_3$-alkoxy)$_2$, –$SO_2$N($C_1$–$C_3$-alkyl)$_2$;
b) –$COOCH_3$, –$COOC_2H_5$, –$COCH_3$, –$COC_2H_5$, –$CON(CH_3)_2$, –$CON(C_2H_5)_2$, Cyano, Nitro, –$SO_2$–$CH_3$, –$SO_2$–$C_2H_5$, –SO–$CH_3$, –P(O)$(OCH_3)_2$, –P(O)$(OC_2H_5)_2$, –$SO_2$–N$(CH_3)_2$, –$SO_2$–N$(C_2H_5)_2$;
c) –$COOCH_3$, –$COCH_3$, –$CON(CH_3)_2$, Cyano, Nitro, –$SO_2$–$CH_3$, –P(O)$(OC_2H_5)_2$, –$SO_2$–N$(CH_3)_2$;
d) –$COCH_3$, CN.

Bei $R_3$:
a) $C_1$–$C_3$-Haloalkyl;
b) $C_1$–$C_2$-Haloalkyl;
c) $CCl_3$, $CCl_2F$, $CCl_2H$, $CClH_2$, $CF_3$, $CF_2H$, $C_2Cl_5$, $CCl_2CHCl_2$;
d) $CCl_3$, $CCl_2F$, $CCl_2CHCl_2$.

Es ergeben sich beispielsweise folgende Kombinationen a bis g dieser Substituententypen untereinander:
a) ($R_1$-a), (X,Y,Z-a), (U,V,W-a), ($R_2$-a), ($R_3$-a)
b) ($R_1$-b), (X,Y,Z-b), (U,V,W-b), ($R_2$-b), ($R_3$-b)
c) ($R_1$-c), (X,Y,Z-c), (U,V,W-c), ($R_2$-c), ($R_3$-c)
d) ($R_1$-d), (X,Y,Z-d), ($R_2$-d), ($R_3$-d)
e) ($R_1$-a), (X,Y,Z-c), (U,V,W-c), ($R_2$-b), ($R_3$-c)
f) ($R_1$-b), (X,Y,Z-c), (U,V,W-c), ($R_2$-d), ($R_3$-d)
g) ($R_1$-d), (X,Y,Z-d), ($R_2$-a), ($R_3$-a)

So sind beispielsweise hierin folgende Wirkstoffgruppen bevorzugt:

a) Verbindungen der Formel I, worin $R_1$ für eines der Fragmente
[X,Y,Z(Phenyl)], [X,Y,Z(Biphenyl-4-yl)],
[X,Y,Z(Biphenyl-3-yl)], [X,Y,Z(Biphenyl-2-yl)],
[U,V,W(2-Pyridyl)], [U,V,W(3-Pyridyl)],
[U,V,W(4-Pyridyl)], [U,V,W(2-Furyl)],
[U,V,W(3-Furyl)], [U,V,W(2-Thienyl)] oder
[U,V,W-3-Thienyl]
steht, wobei X, Y, Z unabhängig voneinander für Wasserstoff, $C_1$–$C_3$-Alkyl, $C_1$–$C_3$-Haloalkyl, Di($C_1$–$C_3$-alkyl)amino, Nitro, Cyano, –COO($C_1$–$C_3$-alkyl), –CON($C_1$–$C_3$-alkyl)$_2$, $C_1$–$C_4$-Alkoxy, $C_1$–$C_4$-Thioalkyl, $C_3$–$C_5$-Alkenyloxy, $C_3$–$C_5$-Haloalkenyloxy, Phenoxy oder Benzyloxy stehen; U, V, W unabhängig voneinander für Wasserstoff, Halogen oder $C_1$–$C_4$-Alkyl stehen; $R_2$ –COO($C_1$–$C_3$-alkyl), –CO–($C_1$–$C_3$-alkyl), –CO–N($C_1$–$C_3$-alkyl)$_2$, Cyano, Nitro, –$SO_2$–($C_1$–$C_3$-alkyl), –P(O)($C_1$–$C_3$-alkoxy)$_2$ oder –$SO_2$–N($C_1$–$C_3$-alkyl)$_2$ bedeutet; und $R_3$ für $C_1$–$C_3$-Haloalkyl steht.

b) Verbindungen der Formel I, worin $R_1$ für eines der Fragmente
[X,Y,Z(Phenyl)], [X,Y,Z(Biphenyl-4-yl)],
[U,V,W(2-Pyridyl)], [U,V,W(3-Pyridyl)],
[U,V,W(2-Furyl)] oder [U,V,W(2-Thienyl)]
steht; wobei X, Y, Z unabhängig voneinander für Wasserstoff, Fluor, Chlor, Brom, Methyl, Ethyl, Trifluormethyl, –N$(CH_3)_2$, –N$(C_2H_5)_2$, –$COOCH_3$, –$COOC_2H_5$, –$CON(CH_3)_2$, $C_1$–$C_4$-Alkoxy, $C_1$–$C_4$-Alkylthio, $C_3$–$C_5$-Alkenyloxy, $C_3$–$C_5$-Haloalkenyloxy, Phenoxy, oder Benzyloxy stehen; U, V, W unabhängig voneinander für Wasserstoff, Chlor, Brom oder $C_1$–$C_2$-Alkyl stehen; $R_2$ –$COOCH_3$, –$COOC_2H_5$, –$COCH_3$, –$COC_2H_5$, –$CON(CH_3)_2$, –$CON(C_2H_5)_2$, Cyano, Nitro, –$SO_2$–$CH_3$, –$SO_2$–$C_2H_5$, –P(O)$(OCH_3)_2$, –SO–$CH_3$, –P(O)$(OC_2H_5)_2$, –$SO_2$–N$(CH_3)_2$ oder –$SO_2$–N$(C_2H_5)_2$ bedeutet; und $R_3$ für $C_1$–$C_2$-Haloalkyl steht.

Innerhalb der Untergruppe b sind insbesondere solche Verbindungen der Formel I bevorzugt, worin $R_1$ für eines der Molekülfragmente
[X,Y,Z(Phenyl)], [U,V,W(2-Pyridyl)],
[U,V,W(3-Pyridyl)], [U,V,W(2-Furyl)] oder
[U,V,W(2-Thienyl)]
steht; wobei X, Y, Z unabhängig voneinander für Fluor, Chlor, Brom, Methyl, Ethyl, Trifluormethyl, $C_1$–$C_3$-Alkoxy oder $C_1$–$C_3$-Alkylthio stehen; U, V, W unabhängig voneinander für Wasserstoff, Chlor, Brom, Methyl oder Ethyl stehen; $R_2$ –$COOCH_3$, $COCH_3$, $NO_2$ oder CN bedeutet; und $R_3$ für $CFCl_2$ oder $CCl_3$ steht.

c) Verbindungen der Formel I, worin $R_1$ für eines der Fragmente [X,Y,Z(Phenyl)] oder [U,V,W(2-Furyl)] steht; wobei X, Y, Z unabhängig voneinander für Wasserstoff, Chlor, Brom, Methyl, Ethyl, Trifluormethyl, –N$(CH_3)_2$, –$COOCH_3$, –$CON(CH_3)_2$, $C_1$–$C_4$-Alkoxy, $C_1$–$C_4$-Alkylthio, $C_3$-Alkenyloxy, Phenoxy oder Benzyloxy stehen; U, V, W unabhängig voneinander Wasserstoff, Chlor, Brom oder $C_1$–$C_2$-Alkyl bedeuten; $R_2$ für –$COOCH_3$, –$COCH_3$, –$CON(CH_3)_2$, Cyano, Nitro, –$SO_2$–$CH_3$, –P(O)$(OC_2H_5)_2$ oder –$SO_2$–N$(CH_3)_2$ steht; und $R_3$ $CCl_3$, $CCl_2F$, $CCl_2H$, $CClH_2$, $CF_3$, $CF_2H$, $C_2Cl_5$ oder $CCl_2CHCl_2$ bedeutet.

d) Verbindungen der Formel I, worin $R_1$ für das Molekülfragment [X,Y,Z(Phenyl)] steht; wobei X, Y, Z unabhängig voneinander für Wasserstoff, Chlor, Brom, Methyl, $C_1$–$C_4$-Alkoxy, $C_1$–$C_4$-Alkylthio oder $C_3$-Alkenyloxy stehen; $R_2$ –$COCH_3$ oder Cyano bedeutet; und $R_3$ für $CCl_3$, $CCl_2F$ oder $CCl_2CHCl_2$ steht.

Im Rahmen der vorliegenden Erfindung sind folgende Einzelsubstanzen besonders bevorzugt:

a) Zwischenprodukte der Formel II', insbesondere aufgrund vorteilhafter Wirkung im Vorrats- und Pflanzenschutz:
3-(2-Methylthiophenyl)-4-cyanopyrrol,
3-(2-Methoxyphenyl)-4-cyanopyrrol

b) Endprodukte der Formel I, insbesondere aufgrund ihrer ausgeprägten fungiziden Eigenschaften:
N-Fluordichlormethylsulfenyl-3-(2-allyloxyphenyl)-4-cyanopyrrol,
N-Fluordichlormethylsulfenyl-3-(2-chlorphenyl)-4-cyanopyrrol,
N-Fluordichlormethylsulfenyl-3-phenyl-4-acetylpyrrol,
N-Fluordichlormethylsulfenyl-3-(2-chlorphenyl)-4-acetylpyrrol,
N-Fluordichlormethylsulfenyl-3-(4-methylphenyl)-4-acetylpyrrol,
N-Fluordichlormethylsulfenyl-3-(3-chlorphenyl)-4-acetylpyrrol,
N-Fluordichlormethylsulfenyl-3-(3-methylphenyl)-4-acetylpyrrol,

N-Fluordichlormethylsulfenyl-3-(3-chlorphenyl)-4-methoxycarbonylpyrrol,
N-Fluordichlormethylsulfenyl-3-(2-chlorphenyl)-4-methoxycarbonylpyrrol,
N-Fluordichlormethylsulfenyl-3-(2-chlorphenyl)-4-nitropyrrol,
N-Fluordichlormethylsulfenyl-3-(2-furyl)-4-cyanopyrrol,
N-Trichlormethyl-3-(2-pyridyl)-4-cyanopyrrol,
N-Fluordichlormethylsulfenyl-3-(2,3-dichlorphenyl)-4-cyanopyrrol.

Die Verbindungen der Formel I werden erfindungsgemäss dadurch hergestellt, dass man ein freies Pyrrol der Formel II

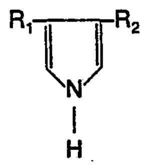 (II)

in Gegenwart einer Base mit einem reaktionsfähigen Säurederivat einer Sulfensäure der Formel III

$$R_3\text{–S–OH} \qquad (III)$$

am Pyrrol-Stickstoff sulfenyliert. Hierbei haben die Substituenten $R_1$, $R_2$ und $R_3$ die unter der allgemeinen Formel I angegebenen Bedeutungen.

Für diese Sulfenylierungsreaktion eignen sich als reaktionsfähige Sulfensäurederivate z.B. die Niederalkylester und bevorzugt die Sulfensäurehalogenide, insbesondere die -chloride und -bromide und von diesen wiederum besonders die -chloride. Niederalkyl steht hierbei für $C_1$–$C_6$-Alkyl.

Als Basen können sowohl organische als auch anorganische Basen mit Erfolg eingesetzt werden. Geeignete anorganische Basen sind z.B. Alkali- und Erdalkalicarbonate, wie Natriumcarbonat, Kaliumcarbonat, Calciumcarbonat usw. Geeignete organische Basen sind z.B. tertiäre Amine wie Trialkylamine (Triethylamin, Methyldiethylamin), N,N-Dimethoxycyclohexylamin, N-Methylpiperidin, N,N-Dimethylanilin oder Pyridine. Bevorzugt werden die Trialkylamine. Vorteilhafterweise wird die Base in stöchiometrischer Menge oder im Überschuss, z.B. bis zu 100% Überschuss, in bezug auf das Pyrrol II eingesetzt. Auch das reaktionsfähige Derivat der Sulfensäure III wird entweder in stöchiometrischer Menge oder im Überschuss zugesetzt.

Die Sulfenylierungs-Reaktion kann in An- oder Abwesenheit, vorzugsweise in Anwesenheit eines reaktionsinerten Lösungsmittels oder Lösungsmittelgemisches, durchgeführt werden. Prinzipiell eignen sich für diese Umsetzung die üblichen organischen Lösungsmittel, sofern sie keine reaktiven Wasserstoffatome enthalten. Geeignet sind beispielsweise aliphatische und aromatische Kohlenwasserstoffe wie Benzol, Toluol, Xylole, Petrolether, halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Ethylenglykol- und Diethylenglykoldiether und -monoether mit je 1–4 C-Atomen in den Alkylteilen, wie Ethylenglykoldimethyl-, -diethyl- und -di-n-butylether, Diethylenglykoldiethyl- und -di-n-butylether, Ethylenglykolmonomethylether und Diethylenglykolmonomethylether; Furan, Dimethoxyethan, Dioxan, Tetrahydrofuran, Anisol; Sulfone wie Dimethylsulfoxid; Ketone wie Aceton, Methylethylketon; Ester wie Ethylacetat, Propylacetat; Butylacetat und Gemische solcher Lösungsmittel untereinander. In einigen Fällen kann auch das Sulfenylierungsreagenz der Formel III selbst als Lösungsmittel fungieren.

Zur Beschleunigung der Reaktionsgeschwindigkeit kann unter Umständen ein Reaktionskatalysator wie 4-Dimethylaminopyridin zugesetzt werden.

Die Sulfenylierungsreaktion wird im allgemeinen bei Temperaturen zwischen −30° bis +100 °C, vorzugsweise −10° bis +20 °C, durchgeführt. Die Reaktionszeiten liegen dann erfahrungsgemäss etwa zwischen 0,5 h bis 20 h. Durch Zugabe eines Reaktionskatalysators erreicht man u.U. jedoch oft eine Verkürzung der Reaktionsdauer auf weniger als 0,5 h.

Die freien Sulfensäuren der Formel III sind bei normalen Temperaturen im allgemeinen relativ instabile Substanzen, die zur Selbstoxidation neigen. Stabil sind dagegen die an sich bekannten Sulfensäurehalogenide, die z.B. durch Halogenierung aus den zugrundeliegenden Merkaptanen oder Dialkyldisulfiden zugänglich sind. Bekannt sind auch Niederalkylsulfensäureester, die beispielsweise durch Reaktion von Sulfensäurechloriden mit Alkalialkoholaten hergestellt werden können. Sie lagern sich in der Wärme in die entsprechenden Sulfoxide um, sind jedoch bei tieferen Temperaturen handhabbar.

Ein Teil der Verbindungen der Pyrrole der Formel III ist aus der Literatur bekannt. So werden z.B. die Herstellungsmethode und die chemischen Eigenschaften von 4-Cyano-3-phenyl-pyrrol in Tetrahedron-Letters No. 52, S. 5337–5340 (1972) beschrieben. Über eine biologische Wirksamkeit der Verbindung wird nicht berichtet.

Unterschiedlich substituierte 3-Phenyl-4-cyanopyrrol-derivate sind aus der Literatur bekannt, so werden beispielsweise Pyrrole der Formel IV

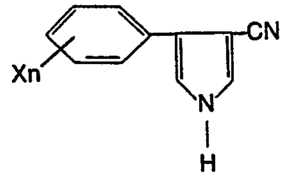 (IV),

worin X ein Halogenatom, eine niedere Alkyl- oder niedere Halogenalkylgruppe bedeutet und n für 0, 1 oder 2 steht, in der DE-OS 2 927 480 als Zwischenprodukte mit geringer fungizider Aktivität beschrieben.

Pyrrol-derivate der Formel II'

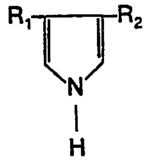

(II'),

worin $R_1$ für eines der Fragmente [X,Y,Z(Phenyl)], [X,Y,Z(Biphenyl)], [U,V,W(Pyridyl)], [U,V,W(Furyl)] oder [U,V,W(Thienyl)] steht, wobei X, Y und Z unabhängig voneinander für Wasserstoff, Halogen, $C_1$–$C_4$-Alkyl, $C_1$–$C_3$-Haloalkyl, Di($C_1$–$C_4$-alkyl)amino, Nitro, Cyano, –COO($C_1$–$C_4$-alkyl), –CON($C_1$–$C_4$-alkyl)$_2$ oder die Gruppe –E–$R_4$ bedeuten, wobei E für –O–, –S–, –SO– oder –$SO_2$– steht, $R_4$ unsubstituiertes oder durch $C_1$–$C_4$-Alkoxy substituiertes $C_1$–$C_6$-Alkyl, unsubstituiertes oder durch Halogen substituiertes $C_3$–$C_5$-Alkenyl, unsubstituiertes oder durch Halogen oder Hydroxy substituiertes $C_3$–$C_5$-Alkinyl; [X,Y,Z(Phenyl)] oder –$CH_2$–[X,Y,Z-(Phenyl)] bedeutet, U, V, W unabhängig voneinander für Wasserstoff, Halogen oder $C_1$–$C_4$-Alkyl stehen; $R_2$ für –COO($C_1$–$C_6$-alkyl), –CO–($C_1$–$C_6$-alkyl, –CO–N($C_1$–$C_6$-alkyl)$_2$, Nitro, –$SO_2$–($C_1$–$C_6$-alkyl), –P(O)($C_1$–$C_6$-alkoxy)$_2$, –SO–($C_1$–$C_6$-alkyl) oder für –$SO_2$–N($C_1$–$C_3$-alkyl)$_2$ steht, und in den Fällen, in denen $R_1$ für eines der Fragmente [X,Y,Z(Biphenyl)], [U,V,W(Pyridyl)], [U,V,W(Furyl)] oder [U,V,W-(Thienyl)] steht, $R_2$ zusätzlich die Bedeutung Cyano hat; sind neu. Sie stellen besonders entwickelte Zwischenprodukte zur Herstellung der wertvollen Wirkstoffe der Formel I dar. Aufgrund ihrer strukturellen Beschaffenheit lassen sie sich leicht – durch N-Sulfenylierungsreaktion – in die Verbindungen der Formel I überführen. Darüberhinaus zeigen Verbindungen der Formel II' fungizide Aktivität gegenüber wichtigen Schadpilzen im Pflanzenschutz, sowie hervorragende Vorratschutzwirkung. Die neuen Verbindungen der Formel II' sind daher einschliesslich ihrer Herstellungsverfahren und Verwendung ein Bestandteil dieser Erfindung.

Die Verbindungen der Formel II und somit auch diejenigen der Formel II' lassen sich in alkalischem Medium durch cyclisierende Michael-Addition einer Verbindung der Formel V mit p-Tolylsulfonylmethylisocyanid unter Abspaltung von p-Toluolsulfinsäure bzw. ihres Salzes herstellen.

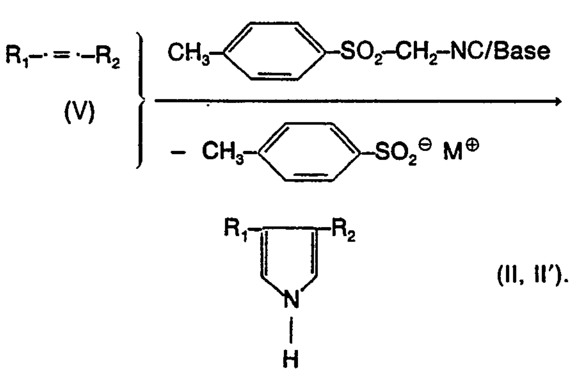


(V)

(II, II').

Dabei sind die Substituenten $R_1$ und $R_2$ wie oben definiert.

Die p-Tolylsulfonyl-Gruppe steht hier und im folgenden stellvertretend für eine ganze Reihe von Gruppen, die in der Lage sind, die Methylengruppe im Methylisocyanid-Rest für eine Reaktion im Sinne einer Michael-Addition zu aktivieren. Weitere bevorzugte Beispiele für derartig aktivierende Gruppen sind Benzolsulfonyl-, p-Chlorbenzolsulfonyl, Niederalkylsulfonyl wie Mesyl.

Die Cycloaddition wird vorteilhafterweise in Gegenwart einer nicht nukleophilen Base durchgeführt. Als Basen eignen sich Alkalimetallhydride wie Natriumhydrid oder Alkali- bzw. Erdalkalicarbonate, wie $Na_2CO_3$, $K_2CO_3$ oder Alkalialkoholate, wie $(CH_3)_3CO^{\ominus}K^{\oplus}$ und andere. Die Base wird vorteilhafterweise in mindestens äquimolarer Menge, bezogen auf die Ausgangsstoffe, eingesetzt.

Wie bei allen Umsetzungen, so ist es auch hier zweckmässig, die Reaktion in einem reaktionsinerten Lösungsmittel durchzuführen. Für die beschriebene Cycloaddition kommen beispielsweise folgende, bevorzugt wasserfreie Lösungsmittel in Frage: Aromatische und aliphatische Kohlenwasserstoffe wie Benzol, Toluol, Xylole, Petrolether, Ligroin, Cyclohexan; Ether und etherartige Verbindungen wie Dialkylether (Diethylether, Diisopropylether, t-Butylmethylether usw.), Dimethoxymethan, Dioxan, Tetrahydrofuran, Anisol; Sulfone wie Dimethylsulfoxid; Dimethylformamid und Gemische solcher Lösungsmittel untereinander.

Die Cycloaddition wird im allgemeinen bei Temperaturen von −30° bis +120°C, bevorzugt bei −30° bis +50°C bzw. am Siedepunkt des Lösungsmittels oder Lösungsmittelgemisches, durchgeführt.

Bei der Wahl geeigneter Basen lässt sich die Cycloaddition vorteilhafterweise auch in wässrigem Medium ausführen. Als Basen eignen sich in diesen Fällen wasserlösliche anorganische und organische Basen, insbesondere Alkalihydroxide wie LiOH, NaOH oder KOH und Ammoniumbasen, z.B. Tetraalkylammoniumhydroxide wie $(CH_3)_4NOH$. Die Base wird in mindestens äquimolarer Menge, bezogen auf die Ausgangsstoffe, eingesetzt. Bei der Verwendung wässriger Basen führt man die Reaktion vorteilhafterweise in einem heterogenen Zweiphasensystem durch.

Für die organische, mit Wasser nicht mischbare Phase kommen z.B. folgende Lösungsmittel in Frage: Aliphatische und aromatische Kohlenwasserstoffe, wie Pentan, Hexan, Cyclohexan, Petrolether, Ligroin, Benzol, Toluol, Xylole usw., halogenierte Kohlenwasserstoffe, wie Dichlormethan, Chloroform, Tetrachlorkohlenstoff, Ethylendichlorid, 1,2-Dichlorethan, Tetrachlorethylen usw., oder aliphatische Ether, wie Diethylether, Diisopropylether, t-Butylmethylether usw.

Zur Beschleunigung der Reaktionsgeschwindigkeit kann bei dieser Durchführungsart die Gegenwart eines Phasentransfer-Katalysators von Vorteil sein. Beispiele derartiger Katalysatoren sind: Tetraalkylammoniumhalogenide, -hydrogensulfate oder -hydroxide wie Tetrabutylammoniumchlorid, -bromid, -jodid; Triethylbenzylammoniumchlorid, -bromid; Tetrapropylammoniumchlorid, -bromid-, -jodid usw. Als Phasentransfer-Katalysatoren kommen auch Phosphonium-Salze in Betracht.

Die phasentransfer-katalysierte Cycloaddition kann bei Temperaturen von 0° bis +80 °C, vorzugsweise +10° bis +50 °C bzw. beim Siedepunkt des Lösungsmittelgemisches, durchgeführt werden.

Die Cycloaddition kann in den beschriebenen Ausführungsarten bei Normaldruck erfolgen; die Reaktionsdauer beträgt im allgemeinen 1 bis 16 Stunden, bei Phasentransferkatalyse 0,5 bis 10 Stunden.

Es sind bereits verschiedentlich substituierte Fünfringheterocyclen, auch solche mit einem Haloalkylsulfenyl-Substituenten, als fungizide Wirkstoffe beschrieben (vgl. R. Wegler: Chemie der Pflanzenschutz- und Schädlingsbekämpfungsmittel, Bd. 4, 1977, S. 190–195, 220; US-PS 2 858 462, CA-689 434, DE-A 2 903 458 und DE-A-2 927 480), die jedoch bisher nicht immer die Bedürfnisse der Praxis vor allem hinsichtlich der Wirkungsbreite und Verträglichkeit im erforderlichen Masse befriedigen konnten.

Es wurde überraschend festgestellt, dass Verbindungen der Formel I ein für praktische Bedürfnisse sehr günstiges Mikrobizid-Spektrum gegen phytopathogene Pilze und Bakterien aufweisen. Sie können sowohl im Pflanzenschutz zur Bekämpfung schädlicher Mikroorganismen an Kulturpflanzen, als auch im Vorratsschutz zur Konservierung verderblicher Waren eingesetzt werden. Verbindungen der Formel I besitzen sehr vorteilhafte kurative, systemische und insbesondere präventive Eigenschaften und lassen sich zum Schutz von zahlreichen Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Mikroorganismen eingedämmt oder vernichtet werden, wobei auch später zuwachsende Pflanzenteile von derartigen Mikroorganismen verschont bleiben. Eine entsprechende Aktivität zeigen auch die Zwischenprodukte der Formel II'.

Die erfindungsgemässen Wirkstoffe sind beispielsweise gegen die den folgenden Klassen angehörenden phytopathogenen Pilze wirksam: Ascomycetes z.B. Erysiphe, Sclerotinia, Fusarium, Monilinia, Helminthosporium; Basidiomycetes wie z.B. Puccinia, Tilletia, Rhizoctonia; sowie die der Klasse der Phycomycetes angehörenden Oomycetes wie Phytophthora. Als Pflanzenschutzmittel können die Verbindungen der Formel I mit besonders gutem Erfolg gegen wichtige Schadpilze aus der Familie der Fungi imperfecti eingesetzt werden, so z.B. gegen Cercospora, Piricularia und vor allem gegen Botrytis. Botrytis spp. (B. Cinerea, B. allii) stellen mit der Grauschimmelfäule an Reben, Erdbeeren, Äpfeln, Zwiebeln und anderen Obst- und Gemüsesorten einen bedeutenden wirtschaftlichen Schadfaktor dar. Darüberhinaus lassen sich Verbindungen der Formel I erfolgreich zum Schutz verderblicher Waren pflanzlicher oder tierischer Herkunft einsetzen. Sie bekämpfen Schimmelpilze wie Penicillium, Aspergillus, Rhizopus, Fusarium, Helminthosporium, Nigrospora und Alternaria sowie Bakterien wie Buttersäurebakterien und Hefen wie Candida. Diese Wirk-Substanzen zeigen ferner hervorragende Wirkung gegen Boden- und Samenbürtige Pilze.

Als Pflanzenschutzmittel besitzen die Verbindungen der Formel I ein für die praktische Anwendung in der Landwirtschaft sehr günstiges Wirkungsspektrum zum Schutze von Kulturpflanzen, ohne diese durch unerwünschte Nebenwirkungen nachteilig zu beeinflussen.

Sie können somit auch als Beizmittel zur Behandlung von Saatgut (Früchte, Knollen, Körner) und Pflanzenstecklingen zum Schutz vor Pilzinfektionen sowie gegen im Erdboden auftretende phytopathogene Pilze eingesetzt werden.

Die Erfindung betrifft somit auch mikrobizide Mittel sowie die Verwendung der Verbindungen der Formel I zur Bekämpfung phytopathogener Mikroorganismen, insbesondere pflanzenschädigender Pilze bzw. die präventive Verhütung eines Befalls an Pflanzen und an Vorräten pflanzlicher oder tierischer Herkunft.

Darüberhinaus schliesst die vorliegende Erfindung auch die Herstellung (agro)chemischer Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen oder Vorratsgütern, das sich durch Applikation der Verbindungen der Formel I bzw. der neuen Mittel auf die Pflanzen, Pflanzenteile, deren Standort oder das Substrat kennzeichnet.

Als Zielkulturen für den Pflanzenschutz gelten im Rahmen dieser Erfindung beispielsweise folgende Pflanzenarten: Getreide: (Weizen, Gerste, Roggen, Hafer, Reis, Sorghum und Verwandte); Rüben: (Zucker- und Futterrüben); Kern-, Stein- und Beerenobst: (Äpfel, Birnen, Pflaumen, Pfirsiche, Mandeln, Kirschen, Erd-, Him- und Brombeeren); Hülsenfrüchte: (Bohnen, Linsen, Erbsen, Soja); Ölkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Im Vorratschutz werden die Verbindungen der Formeln I bzw. II' in unveränderter Form oder vorzugsweise zusammen mit den in der Formulierungstechnik üblichen Hilfsmitteln eingesetzt und werden z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen und die Form des Mittels werden den angestrebten Zielen und den gegebenen Verhältnissen angepasst. Günstige Aufwandmengen liegen im allgemeinen bei 0,01 bis höchstens 2 kg Aktivsubstanz pro 100 kg zu schützendes Substrat; sie hängen jedoch ganz wesentlich von der Beschaffenheit (Grösse der Oberfläche, Konsistenz, Feuchtigkeitsgehalt) des Substrats und dessen Umgebungseinflüssen ab.

Unter Lager- und Vorratsgütern sollen im Rahmen vorliegender Erfindung pflanzliche und/oder tierische Naturstoffe und deren Weiterverarbeitungsprodukte verstanden werden, beispielsweise die nachfolgend aufgezählten und aus dem natürlichen Lebenszyklus herausgenommenen Pflanzen, deren Pflanzenteile (Stengel, Blätter, Knollen, Samen, Früchte, Körner), die im frisch geernteten Zustand oder in weiterverarbeitbarer Form vorliegen (vorgetrocknet, befeuchtet, zerkleinert, gemahlen, geröstet). Als Beispiele, die keinen das Anwendungsgebiet limitierenden Charakter im Rahmen dieser Erfindung besitzen, seien folgende Ertragsgüter genannt: Getreide (wie Weizen, Gerste, Roggen, Hafer, Reis, Sorghum und Verwandte); Rüben (wie Möhren, Zucker- und Futterrüben); Kern-, Stein- und Beerenobst (wie Äpfel, Birnen, Pflaumen, Pfirsiche, Mandeln, Kirschen, Erd-, Him- und Brombeeren); Hülsenfrüchte (wie Bohnen, Linsen, Erbsen, Soja); Ölkulturen (wie Raps, Senf, Mohn, Oliven, Sonnenblumen, Kokos, Rizinus, Kakao, Erdnüsse); Gurkengewächse (wie Kürbis, Gurken, Melonen); Fasergewächse (wie Baumwolle, Flachs, Hanf, Jute, Nessel); Citrusfrüchte; Gemüsesorten (wie Spinat, Salat, Spargel, Kohlarten, Zwiebeln, Tomaten, Kartoffeln, Paprika); Lorbeergewächse (wie Avocado, Cinnamonum, Kampfer) oder Mais, Tabak, Nüsse, Kaffee, Zuckerrohr, Tee, Weintrauben, Kastanien, Hopfen, Bananen, Gras und Heu.

Als Naturprodukte tierischer Herkunft seien insbesondere getrocknete Fleisch- und Fischverarbeitungsprodukte wie Trockenfleisch, Trockenfisch, Fleischkonzentrate, Knochenmehl, Fischmehl und Tiertrockenfutter genannt.

Das behandelte Vorratsgut wird durch Behandlung mit Verbindungen der Formel I nachhaltig vor dem Befall durch Schimmelpilze und andere unerwünschte Mikroorganismen geschützt. Dadurch wird die Bildung toxischer und zum Teil karzinogener Schimmelpilze (Aflatoxine und Ochratoxine) unterbunden, das Gut vor dem Verderben bewahrt und dessen Qualität für längere Zeit aufrechterhalten. Das erfindungsgemässe Verfahren kann auf alle trockenen und feuchten Vorrats- und Lagergüter angewendet werden, die gegen Mikroorganismen, wie Hefen, Bakterien und insbesondere Schimmelpilze anfällig sind.

Ein bevorzugtes Verfahren zum Aufbringen des Wirkstoffes besteht in einem Besprühen oder Benetzen des Substrats mit einer flüssigen Aufbereitung oder im Vermischen des Substrats mit einer festen Aufbereitung der Aktivsubstanz. Das beschriebene Konservierungs-Verfahren ist ein Teil der vorliegenden Erfindung.

Wirkstoffe der Formel I werden üblicherweise in Form von Zusammensetzungen verwendet und können gleichzeitig oder nacheinander mit weiteren Wirkstoffen auf die zu behandelnde Fläche, Pflanze oder Substrat gegeben werden. Diese weiteren Wirkstoffe können sowohl Düngemittel, Spurenelement-Vermittler oder andere des Pflanzenwachstum beeinflussende Präparate sein. Es können aber auch selektive Herbizide, Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeignete Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln. Besonders vorteilhafte Zusatzstoffe sind Phospolipide.

Ein bevorzugtes Verfahren zum Aufbringen eines Wirkstoffes der Formel I bzw. eines (argo)chemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z. B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl, Sonnenblumenöl oder Sojaöl; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht adsorptive Trägermaterialien z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände, wie z. B. Korkmehl oder Sägemehl, verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$–$C_{22}$), wie z. B. die Na- oder K-Salze der Öl- oder Stearinsäure, oder von natürlichen Fettsäuregemischen, die z. B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8–22 C-Atomen. Alkylarylsulfonate sind z. B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14-ethylenoxidadduktes) in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 g Propylenglykolethergruppen enthaltenden Polyethylenoxidaddukte an Polypropylenglykol, Ethylendiaminopolypropylenglykol und Alkypolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z. B. das Stearyltrimethylammoniumchlorid oder das Benzyldi(2-chlorethyl)ethylammoniumbromid. Auf dem Vorratsschutzgebiet werden die für die menschliche und tierische Ernährung unbedenklichen Zuschlagstoffe bevorzugt.

Die in der Formulierungstechnik gebräuchlichen Tenside sind u. a. in folgenden Publikationen beschrieben:

«Mc Cutcheon's Detergents and Emulsifiers Annual» BC
Publishing Corp., Ringwood New Jersey, 1980
Sisely and Wood, «Encyclopedia of Surgace Active Agents»,
Chemical Publishing Co., Inc. New York, 1980.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige (agro)chemische Mittel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Darüberhinaus werden folgende Symbole verwendet: h = Stunde; d = Tag; min = Minute; RT = Raumtemperatur; N = Normalität; abs = absolut, wasserfrei; DMSO = Dimethylsulfoxid; DMF = Dimethylformamid. Druckangaben erfolgen in Millibar mb, oder Bar b. THF steht für Tetrahydrofuran.

Herstellungsbeispiele
d) Ausgangsprodukte

Beispiel a:
Herstellung von

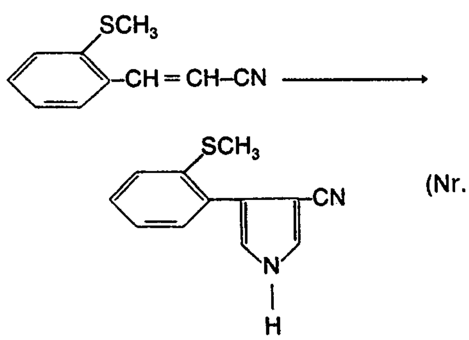

(Nr. 24)

3-(2-Methylthiophenyl)-4-cyanopyrrol

Zu einer Lösung von 158 g (1,4 Mol) Kalium-tert.-butylat in 400 ml THF lässt man bei −13° bis +8° langsam eine Lösung von 123 g (0,7 Mol) E/Z-3-(2-Thiomethylphenyl)-propennitril und 192 g (0,98 Mol) p-Toluolsulfonylmethylisocyanid in 800 ml THF zutropfen. Anschliessend wird das Gemisch noch 2,25 Stunden bei RT gerührt, dann auf 3 Liter Eiswasser gegossen und zweimal mit Essigsäureethylester extrahiert. Die vereinigten Extrakte werden viermal mit halbgesättigter Natriumchloridlösung gewaschen, über Natriumsulfat getrocknet, mittels Kieselgel und Aktivkohle entfärbt, filtriert udn eingeengt. Der Rückstand wird durch Zugabe von Dichlormethan/Petrolether zur Kristallisation gebracht. Ausbeute 95 g beigefarbener Kristalle. Fp. 121–124°. Durch Aufarbeitung der Mutterlauge werden weitere 21 g der Substanz erhalten. Durch mehrfache Umkristallisation aus Dichlormethan/Petrolether erhält man ein fast farbloses Produkt mit Fp. 128–134°.

Beispiel b:
Herstellung von

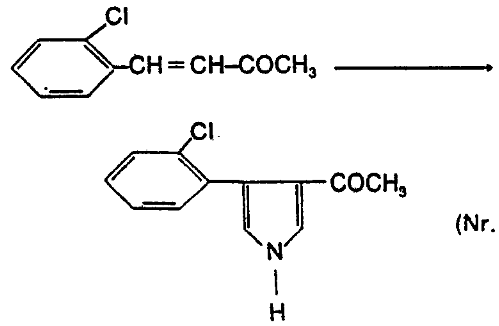

(Nr. 49)

3-(2-Chlorphenyl)-4-acetylpyrrol

Zu 48 g (0,43 Mol) Kalium-tert.butylat in 100 ml THF lässt man bei −10° bis 30° langsam eine Lösung von 60 g (0,33 Mol) E/Z-4-(2-Chlorphenyl)-3-buten-2-on und 75 g (0,38 Mol) p-Toluolsulfonylmethylisocyanid in 400 ml THF zutropfen. Anschliessend wird das Gemisch noch 2,5 Stunden bei 5° bis 10° gerührt, dann auf RT erwärmt, auf 2 Liter Eiswasser gegossen und zweimal mit Essigsäureethylester extrahiert. Die vereinigten Extrakte werden viermal mit halbgesättigter Natriumchlorid-Lösung gewaschen, über Natriumsulfat getrocknet, mit Kieselgel und Bleicherde entfärbt, filtriert und eingeengt. Der Rückstand wird in Dichlormethan unter Rückfluss digeriert, abgekühlt und filtriert. Ausbeute 40 g beigefarbener Kristalle. Fp. 198–201°.

Beispiel c:
Herstellung von

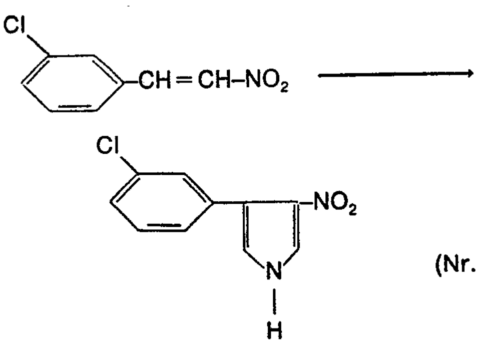

(Nr. 41)

3-(3-Chlorphenyl)-4-nitropyrrol

1,6 g (0,036 Mol) einer ca. 55%igen Natriumhydrid-Dispersion in Mineralöl werden unter Stickstoffatmosphäre zweimal mit Petroläther digeriert und dann mit 50 ml Diethylether versetzt. Zu dieser Mischung lässt man unter kräftigem Rühren eine Lösung von 5,5 g (0,03 Mol) 2-(3-Chlorphenyl)-1-nitroethen und 5,9 g (0,03 Mol) p-Toluolsulfonylmethylisocyanid in 20 ml DMSO und 40 ml Diethylether so zutropfen, dass das Reaktionsgemisch ständig unter Rückfluss siedet. Nach Abklingen der exothermen Reaktion wird noch 15 Min. bei RT weitergerührt. Anschliessend wird das Gemisch zuerst vorsichtig mit Eiswasser und dann mit gesättigter Natriumchlorid-Lösung versetzt und zweimal mit Essigsäureethylester extrahiert. Die vereinigten Extrakte werden viermal mit

halbgesättigter Natriumchlorid-Lösung gewaschen, über Natriumsulfat getrocknet, filtriert und eingeengt. Der Rückstand wird aus Dichlormethan/Petrolether umkristallisiert und führt zu gelben Kristallen mit Fp. 133–135°.

Beispiel d:
Herstellung von

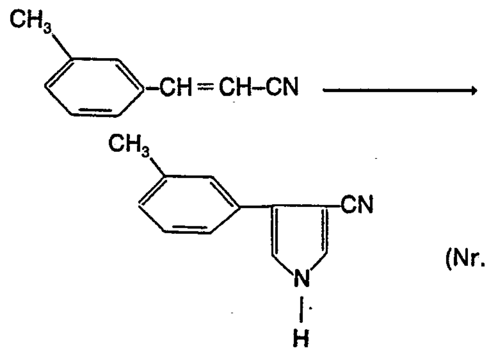

(Nr. 10)

3-(3-Methylphenyl)-4-cyanopyrrol

6,1 g Natriumhydrid-Dispersion (55%-ig in Mineralöl) werden mit Petrolether gewaschen und mit 60 ml Dimethoxyethan und 10 ml DMSO versetzt. Unter Rühren lässt man dazu bei −25° bis −20° langsam eine Lösung von 14,2 g 3-Methylzimtsäurenitril und 21,5 g p-Toluolsulfonylmethylisocyanid in 60 ml Dimethoxyethan und 10 ml DMSO zutropfen, rührt noch 1,5 Stunden bei auftauendem Eisbad, giesst das Gemisch auf Eis und extrahiert zweimal mit Essigsäureethylester. Die organische Phase wird mit wässriger Natriumchloridlösung gewaschen, über Natriumsulfat getrocknet, filtriert und eingeengt. Es werden 19 g eines viskosen Öls erhalten, das nach Umkristallisation aus Dichlormethan/Petroloeumbenzin kristallines 3-(3-Methylphenyl)-4-cyanopyrrol liefert. Fp. 109–111°.

Beispiel e:
Herstellung von

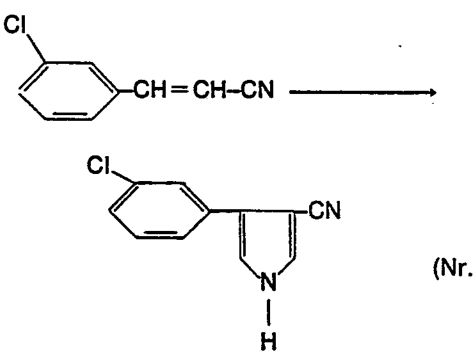

(Nr. 1)

3-(3-Chlorphenyl)-4-cyanopyrrol

a) 10,5 g Natriumhydrid-Dispersion (55%-ig in Mineralöl) werden mit Petrolether gewaschen und anschliessend mit 120 ml THF versetzt. Danach lässt man bei −25° bis −20° eine Lösung aus 30,7 g 3-Chlorzimtsäurenitril und 41 g p-Toluolsulfonylmethylisocyanid in 180 ml THF und 20 ml DMSO zutropfen und rührt das Gemisch noch 2 Stunden bei aufgetautem Eisbad. Anschliessend wird die Mischung auf Eis gegossen, zweimal mit Essigsäureethylester extrahiert; die organische Phase mehrmals mit wässriger Natriumchloridlösung gewaschen, über Natriumsulfat getrocknet, filtriert und das Filtrat eingeengt. Umkristallisation des festen Rückstandes aus Dichlormethan lieferte kristallines 3-(3-Chlorphenyl)-4-cyanopyrrol. Fp. 138–140°.

Beispiel f:
Herstellung von

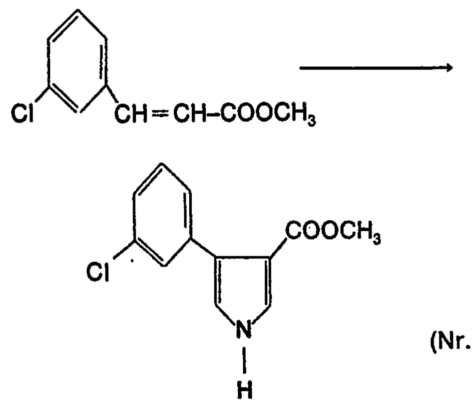

(Nr. 40)

3-(3-Chlorphenyl)-4-methyloxycarbonylpyrrol

Zu 400 ml THF werden unter Rühren zwischen 0° und 10° aus zwei Tropftrichtern gleichzeitig je eine Lösung von 136 g E/Z-3-(3-Chlorphenyl)propensäuremethylester und 155 g p-Toluolsulfonylmethylisocyanid in 500 ml THF und von 109 g Kalium-tert.butylat in 700 ml THF getropft. Man rührt noch 1½ Stunden bei 0°–5° und ½ Stunde bei 25°, giesst das Gemisch auf Eis und extrahiert zweimal mit Essigsäureethylester. Die Extrakte werden viermal mit halbgesättigter Natriumchlorid-Lösung gewaschen, über Natriumsulfat getrocknet, mittels Kieselgel und Aktivkohle entfärbt, filtriert und eingeengt. Der Rückstand wird mit Dichlormethan unter Rückfluss digeriert, das Gemisch über Nacht bei −18° stehengelassen und filtriert. Man erhält 131 g farbloses Produkt vom Fp. 187–189°.

Beispiel g:
Herstellung von

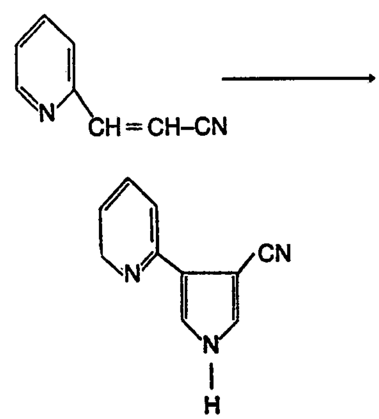

(Nr. 63)

3-(2-Pyridyl)-4-cyanopyrrol

Zu 200 ml THF werden unter Rühren aus zwei Tropftrichtern zwischen −10° und 0° gleichzeitig je eine Lösung von 128,8 g 3-(2-Pyridyl)-propen-2-

nitrit und 200 g p-Toluolsulfonylmethylisocyanid in 1500 ml THF und von 140 g Kalium-tert.-butylat in 1500 ml THF getropft. Man rührt noch 1 Stunde bei 0° bis 5° und 1 Stunde bei 25°, giesst das Gemisch auf Eis und extrahiert zweimal mit Essigsäureethylester. Die organische Phase wird mit wässriger Natriumchloridlösung gewaschen, über Natriumsulfat getrocknet, mit Kieselgel und Aktivkohle verrührt, filtriert und eingeengt. Der dunkle Rückstand wird aus Dichlormethan bei −20° kristallisiert. Es werden 75,5 g braune Kristalle mit Fp. 148–151° erhalten.

Auf analoge Weise lassen sich alle Zwischenprodukte auch die nachfolgenden herstellen; hierbei sind die mit Stern (*) gekennzeichneten Substanzen 8, 9, 11, 13 bis 76 neu. Sie zeigen teilweise pflanzenfungizide Aktivität. Insbesondere die Verbindungen Nr. 16 und 24 sind hoch aktiv gegen Deuteromyceten, insbesondere Botrytispilze sowie gegen schädliche Mikroorganismen im Vorratsschutz. Diese neuen Verbindungen sind ein wichtiger Teil der vorliegenden Erfindung.

Tabelle der Zwischenprodukte der Formel

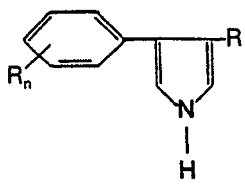

| Verb. Nr. | $R_n$ | R | Fp. [°C] |
|---|---|---|---|
| 1 | 3–Cl | CN | 138–140° |
| 2 | 2,4–$Cl_2$ | CN | 150–152° |
| 3 | 4–Cl | CN | 153–155° |
| 4 | 2–Cl | CN | 136–138° |
| 5 | 4–F | CN | 137–138° |
| 6 | 3–F | CN | 138–139° |
| 7 | 3–Br | CN | 132–134° |
| 8* | 4–$N(CH_3)_2$ | CN | 180–182° |
| 9* | 3–$N(CH_3)_2$ | CN | 144–146° |
| 10 | 3–$CH_3$ | CN | 109–111° |
| 11* | 3–$NO_2$ | CN | 232–234° |
| 12 | 3–$CF_3$ | CN | 87–89° |
| 13* | 3–$OC_6H_5$ | CN | 124–126° |
| 14* | 3–$OCH_3$ | CN | 125–128° |
| 15* | 3–$OCHF_2$ | CN | 95–97° |
| 16* | 2–$OCH_3$ | CN | 135–136° |
| 17* | 2–$OCHF_2$ | CN | 105–107° |
| 18* | 2–$OC_2H_5$ | CN | 134–135° |
| 19* | 2–$OCH(CH_3)_2$ | CN | 80–81° |
| 20* | 2–$OCH_2CH{=}CH_2$ | CN | 83–86° |
| 21* | 2–$OCH_2C_6H_5$ | CN | 91–93° |
| 23* | 3–$SCH_3$ | CN | 115–117° |
| 24* | 2–$SCH_3$ | CN | 128–134° |
| 25* | 2–SO–$CH_3$ | CN | 168–171° |
| 26* | 2–$SO_2$–$CH_3$ | CN | 150–152° |
| 27* | 2–$SC_2H_5$ | CN | 121–123° |
| 28* | 2–SO–$C_2H_5$ | CN | 144–145° |
| 29* | 2–$SO_2$–$C_2H_5$ | CN | 141–143° |
| 30* | 2–S–$CH(CH_3)_2$ | CN | 121–123° |
| 31* | 2–$SO_2$–$CH(CH_3)_2$ | CN | 139–141° |
| 32* | 2–S–$C_4H_9$–n | CN | 61–65° |
| 33* | 2–SO–$C_4H_9$–n | CN | 120–123° |
| 34* | 2–$SO_2C_4H_9$–n | CN | 151–152° |
| 35* | 2–$COOC_2H_5$ | CN | 130–132° |
| 36* | 3–C≡C–$C(OH)(CH_3)_2$ | CN | Oel |
| 37* | 3–C≡C–H | CN | 132–134° |
| 38* | 2–C≡C–$C(OH)(CH_3)_2$ | CN | 140–143° |
| 39* | 2–C≡C–H | CN | 109–115° |
| 40* | 3–Cl | CO–$OCH_3$ | 187–189° |

| Verb. Nr. | $R_n$ | R | Fp. [°C] |
|---|---|---|---|
| 41* | 3–Cl | $NO_2$ | 133–135° |
| 42* | 2–Cl | $NO_2$ | 137–139° |
| 43* | 3–Cl | $P(O)(OC_2H_5)_2$ | 102–104° |
| 44* | 3–Cl | $P(O)(OCH_3)_2$ | 120–122° |
| 45* | 2–Cl | $P(O)(OC_2H_5)_2$ | 129–131° |
| 46* | 2–Cl | $P(O)(OCH_3)_2$ | 146–148° |
| 47* | 2,4–$Cl_2$ | $P(O)(OC_2H_5)_2$ | 89–90° |
| 48* | 3–Cl | CO–$CH_3$ | 193–195° |
| 49* | 2–Cl | CO–$CH_3$ | 198–201° |
| 50* | 2,4–$Cl_2$ | CO–$CH_3$ | 184–186° |
| 51* | H | CO–$CH_3$ | 156–158° |
| 52* | 3–$CH_3$ | CO–$CH_3$ | 120–123° |
| 53* | 4–Cl | CO–$CH_3$ | 170–174° |
| 54* | 3,4–$Cl_2$ | CO–$CH_3$ | 162–164° |
| 55* | 4–$OCH_3$ | CO–$CH_3$ | 179–182° |
| 56* | 4–$CH_3$ | CO–$CH_3$ | 181–184° |
| 57* | 3–Cl | $SO_2$–$CH_3$ | 125–127° |
| 58* | 4–Cl | $SO_2$–$CH_3$ | 154–156° |
| 59* | 2,4–$Cl_2$ | $SO_2$–$CH_3$ | 174–177° |
| 60* | 3–$NO_2$ | $SO_2$–$CH_3$ | 133–135° |
| 61* | 2–Cl, 4–$NO_2$ | $SO_2$–$CH_3$ | 197–200° |
| 62* | 2–Cl | $SO_2$–$CH_3$ | 178–180° |
| 63* | 4–$C_6H_5$ | CN | 249–254° |
| 64* | H | CN | 120–123° |
| 65* | H | $SO_2CH_3$ | |

Tabelle der Zwischenprodukte der Formel

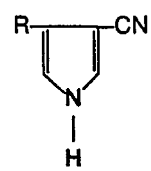

| Verb. No. | R | Fp. [°C] |
|---|---|---|
| 66* | 2–Pyridyl | 148–151° |
| 67* | 3–Pyridyl | 160–162° |
| 68* | 4–Pyridyl | 200–203° |
| 69* | 2–(6–Chlorpyridyl) | 204–206° |
| 70* | 3–(2–Chlorpyridyl) | 217–220° |
| 71* | 2–(3,5–Dichlorpyridyl) | 176–179° |
| 72* | 2–Thienyl | 122–124° |
| 73* | 2–Furyl | 98–100° |
| 75* | 2–(6–Methylthienyl) | 108–110° |
| 76* | 2–(3–Methylthienyl) | 132–137° |

β) Endprodukte

Beispiel H1:
Herstellung von

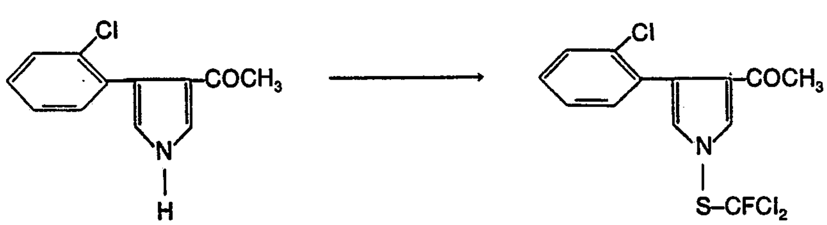

(Nr. 1.24)

N-Fluordichlormethylsulfenyl-3-(2-chlorphenyl)-4-acetylpyrrol

Zu einer Lösung von 11,0 g (0,05 Mol) 3-(2-Chlorphenyl)-4-acetylpyrrol in 100 ml THF lässt man zuerst bei 3° bis 5° eine Lösung von 6 ml (0,0575 Mol) Fluordichlormethylsulfenylchlorid in 20 ml THF und anschliessend bei 5° bis 8° 8 ml (0,0575 Mol) Triethylamin in 20 ml THF zutropfen. Das Gemisch wird dann 16 Stunden bei auftauendem Eisbad gerührt, filtriert und das Filtrat eingeengt. Der Rückstand wird aus Diethylether/Petrolether umkristallisiert. Ausbeute 9,4 g beiger Kristalle mit Fp. 85–87°. Die chromatographische Reinigung (Kieselgel, Dichlormethan/Petrolether 4:1) und Einengung der Mutterlauge liefert nach Umkristallisation aus Diethylether/Petrolether weitere 4,5 g beiger Kristalle mit Fp. 84–86°.

Beispiel H2:

Herstellung von

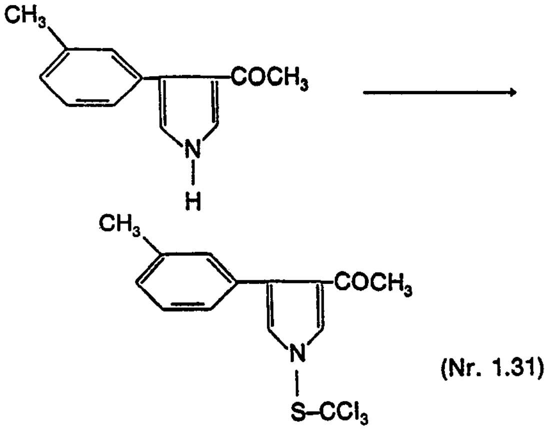

(Nr. 1.31)

N-Trichlormethylsulfenyl-3-(3-methylphenyl)-4-acetylpyrrol

Zu einer Lösung von 6 g (0,03 Mol) 3-(3-Methylphenyl)-4-acetylpyrrol in 100 ml THF lässt man zuerst bei 0° bis 5° 3,9 ml (0,036 Mol) Trichlormethylmercaptan in 30 ml THF und anschliessend bei 5° bis 8° 5 ml (0,036 Mol) Triethylamin in 30 ml THF zutropfen. Das Reaktionsgemisch wird dann 16 Stunden bei auftauendem Eisbad gerührt, filtriert und das Filtrat eingeengt. Der Rückstand wird säulenchromatographisch (Kieselgel, Dichlormethan/Petrolether 2:1) gereinigt und gibt nach Umkristallisation aus Petrolether 5 g farbloser Kristalle mit Fp. 58–59°.

Beispiel H3:

Herstellung von

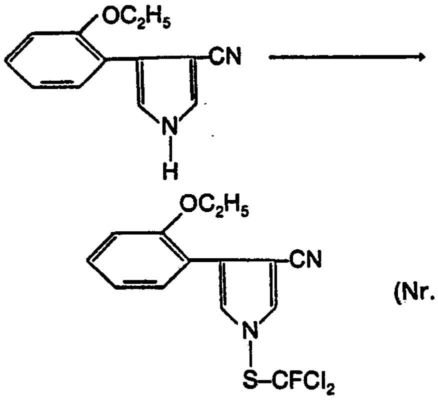

(Nr. 1.15)

N-Fluordichlormethylsulfenyl-3-(2-ethoxyphenyl)-4-cyanopyrrol

Zu einer Lösung von 7,2 g (0,034 Mol) 3-(2-Ethoxyphenyl)-4-cyanopyrrol in 100 ml Essigsäureethylester lässt man zuerst bei 3° bis 7° eine Lösung von 4 ml (0,039 Mol) Fluordichlormethylsulfenylchlorid in 15 ml Essigsäureethylester und anschliessend 5,4 ml (0,039 Mol) Triethylamin in 15 ml Essigsäureethylester zutropfen und rührt das Reaktionsgemisch noch 16 Stunden bei auftauendem Eisbad. Dann wird filtriert und das Filtrat eingeengt. Der Rückstand wird aus Petrolether umkristallisiert. Ausbeute 9,9 g beiger Kristalle mit Fp. 80–83°.

Beispiel H4:

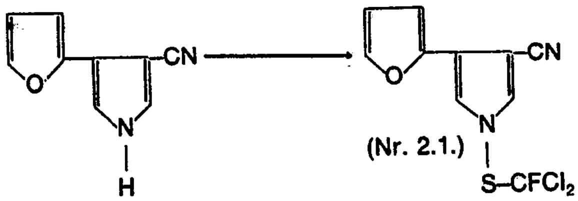

(Nr. 2.1.)

N-Fluordichlormethylsulfenyl-3-(2-furyl)-4 cyanopyrrol

Zu einer Lösung von 5,2 g (0,033 Mol) 3-(2-Furyl)-4-cyanopyrrol in 100 ml THF lässt man zuerst bei 0° bis +5° 3,9 ml (0,037 Mol) Fluordichlormethylsulfenylchlorid in 10 ml THF und anschliessend bei 5° bis 8° 5,2 ml (0,037 Mol) Triethylamin in 10 ml THF zutropfen. Das Reaktionsgemisch wird dann 16 h bei auftauendem Eisbad gerührt, filtriert und das Filtrat eingeengt. Der Rückstand wird säulenchromatographisch (Kieselgel/Dichlormethan/Petrolether 3:1) gereinigt und liefert nach der Umkristallisation aus Petrolether 7 g gelbliche Kristalle mit Fp. 74–76°.

Beispiel H5:

Herstellung von

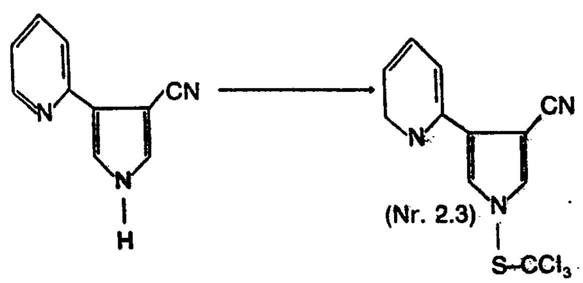

(Nr. 2.3)

N-Trichlormethylsulfenyl-3-(2-pyridyl)-4-cyanopyrrol

Zu einer Lösung von 16,9 g 3-(2-Pyridyl)-4-cyanopyrrol in 200 ml THF lässt man zuerst bei 0° bis 10° eine Lösung von 13,7 und Perchlormethylmercaptan in 20 ml THF und 17,4 ml Triethylamin in 20 ml THF zutropfen. Das Gemisch wird während 16 Stunden bei auftauendem Eisbad gerührt dann eingeengt. Der Rückstand wird zwischen Essigsäureethylester und wässriger Natriumbicarbonat-Lösung verteilt. Die Wasserphase wird abgetrennt, die organische Phase noch einmal mit gesättigter Natriumbicarbonat-Lösung und zweimal mit halbgesättigter Natriumchlorid-Lösung gewaschen, über Natriumsulfat getrocknet, mit Kieselgel und Aktivkohle entfärbt, filtriert und bis zur beginnenden Kristallisation bei 30° Badtemperatur eingeengt. Es wird mit dem gleichen Volumen Petroleumbenzin verdünnt und im Kristallschrank fertig kristallisieren gelassen. 27,5 g farblose Kristalle, die sich zwischen 131° und 140° zersetzen.

Beispiel H6:
Herstellung von

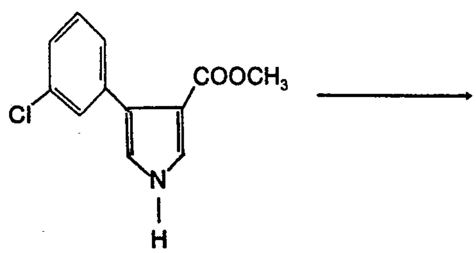

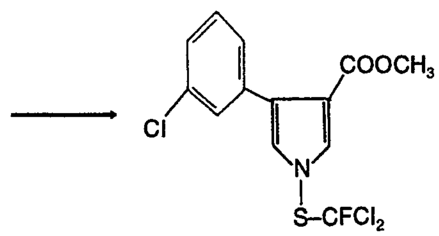

(Nr. 1.34)

N-Fluordichlormethylsulfenyl-3-(3-Chlorphenyl)-4-methyloxycarbonylpyrrol

Zu einer Suspension von 23,6 g 3-(3-Chlorphenyl)-4-methyloxycarbonylpyrrol in 150 ml THF lässt man bei 0° bis 5° zuerst eine Lösung von 20,4 g Fluordichlormethylsulfenylchlorid in 15 ml THF und anschliessend 12,2 g Triethylamin in 15 ml THF zutropfen. Das Gemisch wird während 16 Stunden bei auftauendem Eisbad gerührt, filtriert und das Filtrat eingeengt. Kristallisation aus Essigester/Petroleumbenzin liefert 28 g farbloses Produkt mit Fp. 77–79°.

Auf analoge Weise lassen sich auch die übrigen, im folgenden einzeln aufgezählten Verbindungen der Formel I herstellen.

Tabelle 1: Verbindungen der Formel

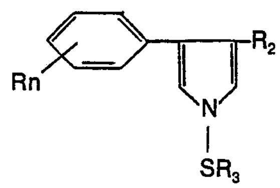

| Verb. Nr. | $R_n$ | $R_3$ | $R_2$ | Fp.[°C] |
|---|---|---|---|---|
| 1.1 | 3–$OCH_3$ | $CCl_2F$ | CN | Oel |
| 1.2 | 2–S–$C_2H_5$ | $CCl_2F$ | CN | 59–60° |
| 1.3 | 2–S–$C_2H_5$ | $CCl_3$ | CN | 90–92° |
| 1.4 | 2–S–$CH(CH_3)_2$ | $CCl_2F$ | CN | 64–66° |
| 1.5 | 2–S–$CH(CH_3)_2$ | $CCl_3$ | CN | 100–102° |
| 1.6 | 2–$SCH_3$ | $CCl_2F$ | CN | 86–87° |
| 1.7 | 2–$SCH_3$ | $CCl_3$ | CN | 125–127° |
| 1.8 | 2–$SC_4H_9$–n | $CCl_2F$ | CN | 44–46° |
| 1.9 | 2–$SC_4H_9$–n | $CCl_3$ | CN | 41–42° |
| 1.10 | 2–$OCH(CH_3)_2$ | $CCl_2F$ | CN | 95–98° |
| 1.11 | 2–$OCH(CH_3)_2$ | $CCl_3$ | CN | 100–102° |
| 1.12 | 2–Benzyloxy | $CCl_2F$ | CN | 127–129° |
| 1.13 | 2–Benzyloxy | $CCl_3$ | CN | 111–113° |
| 1.14 | 2–Br | $CCl_2F$ | CN | 69–71° |
| 1.15 | 2–$OC_2H_5$ | $CCl_2F$ | CN | 86–83° |
| 1.16 | 2–$OC_2H_5$ | $CCl_3$ | CN | 107–110° |
| 1.17 | 2–$OCH_2CH=CH_2$ | $CCl_2F$ | CN | 66–68° |
| 1.18 | 2–$OCH_2CH=CH_2$ | $CCl_3$ | CN | 79–80° |
| 1.19 | 3–Cl | $CCl_2F$ | CN | 87–89° |
| 1.20 | 3–Cl | $CCl_3$ | CN | 81–84° |
| 1.21 | 2–Cl | $CCl_2F$ | CN | 59–61° |
| 1.22 | H | $CCl_2F$ | CO–$CH_3$ | 43–45° |
| 1.23 | H | $CCl_3$ | CO–$CH_3$ | 86–88° |
| 1.24 | 2–Cl | $CCl_2F$ | CO–$CH_3$ | 86–88° |
| 1.25 | 2–Cl | $CCl_3$ | CO–$CH_3$ | 104–109° |
| 1.26 | 4–$CH_3$ | $CCl_2F$ | CO–$CH_3$ | 82–84° |
| 1.27 | 4–$CH_3$ | $CCl_3$ | CO–$CH_3$ | 100–102° |
| 1.28 | 3–Cl | $CCl_2F$ | CO–$CH_3$ | 63–64° |

Tabelle 1 (Fortsetzung)

| Verb. Nr. | $R_n$ | $R_3$ | $R_2$ | Fp.[°C] |
|---|---|---|---|---|
| 1.29 | 3–Cl | $CCl_3$ | CO–$CH_3$ | 82–84° |
| 1.30 | 3–$CH_3$ | $CCl_2F$ | CO–$CH_3$ | $n_D^{52}$: 1.5871 |
| 1.31 | 3–$CH_3$ | $CCl_3$ | CO–$CH_3$ | 58–59° |
| 1.32 | 4–$OCH_3$ | $CCl_2F$ | CO–$CH_3$ | $n_D^{52}$: 1.5927 |
| 1.33 | 4–$OCH_3$ | $CCl_3$ | CO–$CH_3$ | $n_D^{52}$: 1.6098 |
| 1.34 | 3–Cl | $CCl_2F$ | $COOCH_3$ | 77–79° |
| 1.35 | 3–Cl | $CCl_3$ | $COOCH_3$ | 89–90° |
| 1.36 | 3–Cl | $CCl_2F$ | $SO_2CH_3$ | Oel |
| 1.37 | 3–Cl | $CCl_3$ | $SO_2CH_3$ | 104–107° |
| 1.38 | 2,4–$Cl_2$ | $CCl_2F$ | $SO_2CH_3$ | 106–108° |
| 1.39 | 2,4–$Cl_2$ | $CCl_3$ | $SO_2CH_3$ | 111–113° |
| 1.40 | 2–Cl | $CCl_2F$ | $SO_2CH_3$ | 86–88° |
| 1.41 | 2–$OC_2H_5$ | $CCl_2CHCl_2$ | CN | 103° Zers. |
| 1.42 | 2–Cl | $CCl_3$ | CN | 93–95° |
| 1.43 | 2–$OCH_3$ | $CCl_2F$ | CN | 39–42° |
| 1.44 | 2–$OCH_3$ | $CCl_3$ | CN | Harz |
| 1.45 | 2–$OC_3H_7$–n | $CCl_2F$ | CN | 122–127 |
| 1.46 | 2–$OC_3H_7$–n | $CCl_3$ | CN | |
| 1.47 | 2–$OC_4H_9$–n | $CCl_2F$ | CN | |
| 1.48 | 2–$OC_4H_9$–n | $CCl_3$ | CN | |
| 1.49 | 2–$SC_3H_7$–n | $CCl_2F$ | CN | |
| 1.50 | 2–$SC_3H_7$–n | $CCl_3$ | CN | |
| 1.51 | 2–CN | $CCl_2F$ | CN | 112–115° |
| 1.52 | 2–CN | $CCl_3$ | CN | Harz |
| 1.53 | 2,3–$Cl_2$ | $CCl_2F$ | CO–$CH_3$ | |
| 1.54 | 2,3–$Cl_2$ | $CCl_3$ | CO–$CH_3$ | |
| 1.55 | 4–Cl | $CCl_2F$ | CO–$CH_3$ | |
| 1.56 | 4–Cl | $CCl_3$ | CO–$CH_3$ | |
| 1.57 | 2–$SCH_3$ | $CCl_2F$ | CO–$CH_3$ | |
| 1.58 | 2–$SCH_3$ | $CCl_3$ | CO–$CH_3$ | |
| 1.59 | 2–$OCH_3$ | $CCl_2F$ | CO–$CH_3$ | |
| 1.60 | 2–$OCH_3$ | $CCl_3$ | CO–$CH_3$ | |
| 1.61 | 2–CN | $CCl_2F$ | CO–$CH_3$ | |
| 1.62 | 2–CN | $CCl_3$ | CO–$CH_3$ | |
| 1.63 | 4–$N(CH_3)_2$ | $CCl_2F$ | CO–$CH_3$ | |
| 1.64 | 4–$N(CH_3)_2$ | $CCl_3$ | CO–$CH_3$ | |
| 1.65 | 3–$CF_3$ | $CCl_2F$ | CO–$CH_3$ | |
| 1.66 | 3–$CF_3$ | $CCl_3$ | CO–$CH_3$ | |
| 1.67 | 2–Cl | $CCl_2F$ | $COOCH_3$ | Oel |
| 1.68 | 2–Cl | $CCl_3$ | $COOCH_3$ | 83–86° |
| 1.69 | 4–Cl | $CCl_2F$ | $COOCH_3$ | |
| 1.70 | 4–Cl | $CCl_3$ | $COOCH_3$ | |
| 1.71 | 3,4–$Cl_2$ | $CCl_2F$ | $COOCH_3$ | 92–94° |
| 1.72 | 3,4–$Cl_2$ | $CCl_3$ | $COOCH_3$ | 89–90° |
| 1.73 | 4–$CH_3$ | $CCl_2F$ | $COOCH_3$ | |
| 1.74 | 4–$CH_3$ | $CCl_3$ | $COOCH_3$ | |
| 1.75 | 4–$OCH_3$ | $CCl_2F$ | $COOCH_3$ | 97–99° |
| 1.76 | 4–$OCH_3$ | $CCl_3$ | $COOCH_3$ | |
| 1.77 | 2–$OCH_3$ | $CCl_2F$ | $COOCH_3$ | |
| 1.78 | 2–$OCH_3$ | $CCl_3$ | $COOCH_3$ | |
| 1.79 | 2–CN | $CCl_2F$ | $COOCH_3$ | |
| 1.80 | 2–CN | $CCl_3$ | $COOCH_3$ | |
| 1.81 | 2–Cl | $CCl_2F$ | $CON(CH_3)_2$ | |
| 1.82 | 2–Cl | $CCl_3$ | $CON(CH_3)_2$ | |
| 1.83 | 3–Cl | $CCl_2F$ | $CON(CH_3)_2$ | |
| 1.84 | 3–Cl | $CCl_3$ | $CON(CH_3)_2$ | |
| 1.85 | 4–Cl | $CCl_2F$ | $CON(CH_3)_2$ | |
| 1.86 | 4–Cl | $CCl_3$ | $CON(CH_3)_2$ | |
| 1.87 | 4–$CH_3$ | $CCl_2F$ | $CON(CH_3)_2$ | |
| 1.89 | 4–$CH_3$ | $CCl_3$ | $CON(CH_3)_2$ | |

Tabelle 1 (Fortsetzung)

| Verb. Nr. | $R_n$ | $R_3$ | $R_2$ | Fp.[°C] |
|---|---|---|---|---|
| 1.90 | 4–$OCH_3$ | $CCl_2F$ | $CON(CH_3)_2$ | |
| 1.91 | 4–$OCH_3$ | $CCl_3$ | $CON(CH_3)_2$ | |
| 1.92 | 2–$OCH_3$ | $CCl_2F$ | $CON(CH_3)_2$ | |
| 1.93 | 2–$OCH_3$ | $CCl_3$ | $CON(CH_3)_2$ | |
| 1.94 | 2–$OC_2H_5$ | $CCl_2F$ | $CON(CH_3)_2$ | |
| 1.95 | 2–$OC_2H_5$ | $CCl_3$ | $CON(CH_3)_2$ | |
| 1.96 | 2–CN | $CCl_2F$ | $CON(CH_3)_2$ | |
| 1.97 | 2–CN | $CCl_3$ | $CON(CH_3)_2$ | |
| 1.98 | 2–Cl | $CCl_3$ | $SO_2CH_3$ | 118–121° |
| 1.99 | 4–Cl | $CCl_2F$ | $SO_2CH_3$ | |
| 1.100 | 4–Cl | $CCl_3$ | $SO_2CH_3$ | |
| 1.101 | 4–$CH_3$ | $CCl_2F$ | $SO_2CH_3$ | |
| 1.102 | 4–$CH_3$ | $CCl_3$ | $SO_2CH_3$ | |
| 1.103 | 4–$OCH_3$ | $CCl_2F$ | $SO_2CH_3$ | |
| 1.104 | 4–$OCH_3$ | $CCl_3$ | $SO_2CH_3$ | |
| 1.105 | 3,4–$Cl_2$ | $CCl_2F$ | $SO_2CH_3$ | |
| 1.106 | 3,4–$Cl_2$ | $CCl_3$ | $SO_2CH_3$ | |
| 1.107 | 2–$OCH_3$ | $CCl_2F$ | $SO_2CH_3$ | |
| 1.108 | 2–$OCH_3$ | $CCl_3$ | $SO_2CH_3$ | |
| 1.109 | 2–$SC_4H_9$–n | $CCl_2F$ | $SO_2CH_3$ | |
| 1.110 | 2–$SC_4H_9$–n | $CCl_3$ | $SO_2CH_3$ | |
| 1.111 | 2–CN | $CCl_2F$ | $SO_2CH_3$ | |
| 1.112 | 2–CN | $CCl_3$ | $SO_2CH_3$ | |
| 1.113 | 2–Cl | $CCl_2F$ | $SO_2N(CH_3)_2$ | 103–105° |
| 1.114 | 2–Cl | $CCl_3$ | $SO_2N(CH_3)_2$ | |
| 1.115 | 3–Cl | $CCl_2F$ | $SO_2N(CH_3)_2$ | |
| 1.116 | 3–Cl | $CCl_3$ | $SO_2N(CH_3)_2$ | |
| 1.117 | 4–$CH_3$ | $CCl_2F$ | $SO_2N(CH_3)_2$ | |
| 1.118 | 4–$CH_3$ | $CCl_3$ | $SO_2N(CH_3)_2$ | |
| 1.119 | 4–$OCH_3$ | $CCl_2F$ | $SO_2N(CH_3)_2$ | |
| 1.120 | 4–$OCH_3$ | $CCl_3$ | $SO_2N(CH_3)_2$ | |
| 1.121 | 4–Cl | $CCl_2F$ | $SO_2N(CH_3)_2$ | |
| 1.122 | 4–Cl | $CCl_3$ | $SO_2N(CH_3)_2$ | |
| 1.123 | 2–Cl | $CCl_2F$ | $-P(O)(OC_2H_5)_2$ | Oel |
| 1.124 | 2–Cl | $CCl_3$ | $-P(O)(OC_2H_5)_2$ | Oel |
| 1.125 | 3–Cl | $CCl_2F$ | $-P(O)(OC_2H_5)_2$ | |
| 1.126 | 3–Cl | $CCl_3$ | $-P(O)(OC_2H_5)_2$ | |
| 1.127 | 4–Cl | $CCl_2F$ | $-P(O)(OC_2H_5)_2$ | |
| 1.128 | 4–Cl | $CCl_3$ | $-P(O)(OC_2H_5)_2$ | |
| 1.129 | 2–Cl | $-CCl_2F$ | $-NO_2$ | 85–87° |
| 1.130 | 2–Cl | $-CCl_3$ | $-NO_2$ | |
| 1.131 | 3–Cl | $-CCl_2F$ | $-NO_2$ | |
| 1.132 | 3–Cl | $-CCl_3$ | $-NO_2$ | |
| 1.133 | 2–Cl | $-CCl_2F$ | 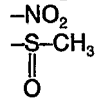 | |
| 1.134 | 2–Cl | $-CCl_3$ | 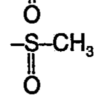 | |
| 1.135 | 2–Cl | $-CCl_2F$ | 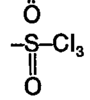 | |
| 1.136 | 2–Cl | $CCl_2$–$CHCl_2$ | CN | 123–125° |
| 1.137 | 4–$C_6H_5$ | $CCl_3$ | CN | 168–175° |
| 1.138 | 4–$C_6H_5$ | $CFCl_2$ | CN | 173–177° |
| 1.139 | H | $CCl_2F$ | CN | 98–98° |
| 1.140 | H | $CCl_3$ | CN | 102–103° |
| 1.141 | H | $CFCl_2$ | $SO_2CH_3$ | |
| 1.142 | 3–Cl | $CCl_2CHCl_2$ | $COOCH_3$ | |
| 1.143 | 2–Br | $CCl_3$ | CN | 114–115 |
| 1.144 | 2–$CH_3$ | $CCl_2F$ | $COCH_3$ | 85–89° |
| 1.145 | 2–$CH_3$ | $CCl_3$ | $COCH_3$ | 94–99° |

Tabelle 1 (Fortsetzung)

| Verb. Nr. | $R_n$ | $R_3$ | $R_2$ | Fp.[°C] |
|---|---|---|---|---|
| 1.146 | 2,3–$Cl_2$ | $CCl_2F$ | CN | 105–107° |
| 1.147 | H | $CCl_2F$ | $SO_2N(CH_3)_2$ | 124–126° |
| 1.148 | H | $CCl_2F$ | $CON(CH_3)_2$ | Oel |
| 1.149 | H | $CCl_3$ | $CON(CH_3)_2$ | 75–79° |
| 1.150 | 2,3–$Cl_2$ | $CCl_2F$ | $COOCH_3$ | 56–61° |

Tabelle 2: Verbindungen der Formel

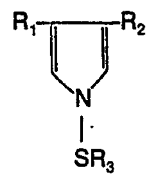

| Verb. Nr. | $R_1$ | $R_2$ | $R_3$ | Fp.[°C] |
|---|---|---|---|---|
| 2.1 | 2–Furyl | CN | $CCl_2F$ | 74–76° |
| 2.2 | 2–Pyridyl | CN | $CCl_2F$ | 92–100° Zers. |
| 2.3 | 2–Pyridyl | CN | $CCl_3$ | 131–140° Zers. |
| 2.4 | 2–Pyridyl | $COOCH_3$ | $CCl_2F$ | |
| 2.5 | 2–Pyridyl | $COOCH_3$ | $CCl_3$ | |
| 2.6 | 2–Pyridyl | $CON(CH_3)_2$ | $CCl_2F$ | |
| 2.7 | 2–Pyridyl | $CON(CH_3)_2$ | $CCl_3$ | |
| 2.8 | 3–(2–Chlorpyridyl | CN | $CCl_2F$ | |
| 2.9 | 3–(2–Chlorpyridyl) | CN | $CCl_3$ | 142–144° |
| 2.10 | 3–(2–Chlorpyridyl) | CO–$CH_3$ | $CCl_2F$ | |
| 2.11 | 3–(2–Chlorpyridyl) | CO–$CH_3$ | $CCl_3$ | |
| 2.12 | 2–(6–Chlorpyridyl) | CN | $CCl_2F$ | |
| 2.13 | 2–(6–Chlorpyridyl) | CN | $CCl_3$ | |
| 2.14 | 2–(6–Chlorpyridyl) | $COOCH_3$ | $CCl_2F$ | |
| 2.15 | 2–(6–Chlorpyridyl) | $COOCH_3$ | $CCl_3$ | |
| 2.16 | 2–Thienyl | CN | $CCl_2F$ | 72–77° |
| 2.17 | 2–Thienyl | CN | $CCl_3$ | 85–89° |
| 2.18 | 2–Thienyl | $CON(CH_3)_2$ | $CCl_2F$ | |
| 2.19 | 2–Thienyl | $CON(CH_3)_2$ | $CCl_3$ | |
| 2.20 | 2–Pyridyl | CN | $CCl_2$–$CHCl_2$ | 103–106° |
| 2.21 | 3–Pyridyl | CN | $CCl_2F$ | 123–126° Zers. |
| 2.22 | 3–Pyridyl | CN | $CCl_3$ | Zers. ab 156° |
| 2.23 | 4–Pyridyl | CN | $CCl_2F$ | Zers. ab 145° |
| 2.24 | 4–Pyridyl | CN | $CCl_3$ | Zers. ab 154° |

Formulierungsbeispiele für flüssige Wirkstoffe der Formel I (% = Gewichtsprozent)

| F1. Emulsions-Konzentrate | a) | b) | c) |
|---|---|---|---|
| Wirkstoff aus den Tabellen | 25% | 40% | 50% |
| Ca–Dodecylbenzolsulfonat | 5% | 8% | 6% |
| Ricinusöl-polyethylen-glykolether (36 Mol Ethylenoxid) | 5% | – | – |
| Tributylphenoyl-polyethy-lenglykolether (30 Mol Ethylenoxid) | – | 12% | 4% |

(Fortsetzung)

| F1. Emulsions-Konzentrate | a) | b) | c) |
|---|---|---|---|
| Cyclohexanon | – | 15% | 20% |
| Xylolgemisch | 65% | 25% | 20% |

Aus solchen Konzentraten können durch Verdünnen mit Wasser Emulsionen jeder gewünschten Konzentration hergestellt werden.

| F2. Lösungen | a) | b) | c) | d) |
|---|---|---|---|---|
| Wirkstoff aus den Tabellen | 80% | 10% | 5% | 95% |
| Ethylenglykol-monomethylether | 20% | – | – | – |
| Polyethylenglykol M G 400 | – | 70% | – | – |
| N-Methyl-2-pyrrolidon | – | 20% | – | – |
| Epoxydiertes Kokosnussöl | – | – | 1% | 5% |
| Benzin (Siedgrenzen 160–190°C) | – | – | 94% | – |
| MG = Molekulargewicht) | | | | |

Die Lösungen sind zur Anwendung in Form kleinster Tropfen geeignet.

| F3. Granulate | a) | b) |
|---|---|---|
| Wirkstoff aus Tabellen | 5% | 10% |
| Kaolin | 94% | – |
| Hochdisperse Kieselsäure | 1% | – |
| Attapulgit | – | 90% |

Der Wirkstoff wird in Methylenchlorid gelöst, auf den Träger aufgesprüht und das Lösungsmittel anschliessend im Vakuum abgedampft.

| F4. Stäubemittel | a) | b) |
|---|---|---|
| Wirkstoff aus den Tabellen | 2% | 5% |
| Hochdisperse Kieselsäure | 1% | 5% |
| Talkum | 97% | – |
| Kaolin | – | 90% |

Durch inniges Vermischen auf Trägerstoffe mit dem Wirkstoff erhält man gebrauchsfertige Stäubemittel.

Formulierungsbeispiele für feste Wirkstoffe der Formel I (% = Gewichtsprozent)

| F5. Spritzpulver | a) | b) | c) |
|---|---|---|---|
| Wirkstoff aus den Tabellen | 25% | 50% | 75% |
| Na-Ligninsulfonat | 5% | 5% | – |
| Na-Laurylsulfat | 3% | – | 5% |
| Na-Diisobutylnaphthalinsulfonat | – | 6% | 10% |
| Octylphenolpolyethylenglykolether (7–8 Mol Ethylenoxid) | – | 2% | – |

(Fortsetzung)

| F5. Spritzpulver | a) | b) | c) |
|---|---|---|---|
| Hochdisperse Kieselsäure | 5% | 10% | 10% |
| Kaolin | 62% | 27% | – |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F6. Emulsions-Konzentrat | |
|---|---|
| Wirkstoff aus den Tabellen | 10% |
| Octylphenolpolyethylenglykolether (4–5 Mol Ethylenoxid) | 3% |
| Ca-Dodecylbenzolsulfonat | 3% |
| Ricinusölpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| F7. Stäubemittel | a) | b) |
|---|---|---|
| Wirkstoff aus den Tabellen | 5% | 8% |
| Talkum | 95% | – |
| Kaolin | – | 92% |

Man erhält anwendungsfertige Stäubemittel, indem der Wirkstoff mit dem Träger vermischt auf einer geeigneten Mühle vermahlen wird.

| F8. Extruder Granulat | |
|---|---|
| Wirkstoff aus den Tabellen | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

| F9. Umhüllungs-Granulat | |
|---|---|
| Wirkstoff aus den Tabellen | 3% |
| Polyethylenglykol (MG 200) | 3% |
| Kaolin | 94% |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

| F10. Suspensions-Konzentrat | |
|---|---|
| Wirkstoff aus den Tabellen | 40% |
| Ethylenglykol | 10% |
| Nonylphenolpolyethylenglykolether (15 Mol Ethylenoxid) | 6% |
| N-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Sowohl die Endprodukte der Formel I als auch die Zwischenprodukte der Formeln II und II' lassen sich gemäss den Beispielen F1 bis F10 formulieren.

Biologische Beispiele:

Beispiel B1:
Wirkung gegen Puccinia graminis auf Weizen
a) Residual-protektive Wirkung

Weizenpflanzen wurden 6 Tage nach der Aussaat mit einer aus Spritzpulver des Wirkstoffes hergestellten Spritzbrühe (0,06% Aktivsubstanz) besprüht. Nach 24 Stunden wurden die behandelten Pflanzen mit einer Uredosporensuspension des Pilzes infiziert. Nach einer Inkubation während 48 Stunden bei 95–100% relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

B) Systemische Wirkung

Zu Weizenpflanzen wurde 5 Tage nach der Aussaat eine aus Spritzpulver des Wirkstoffes hergestellte Spritzbrühe gegossen (0,006% Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95–100% relativer Luftfeuchtigkeit und ca. 20 °C wurden die infizierten Pflanzen in einem Gewächshaus bei ca. 22 °C aufgestellt. Die Beurteilung der Rostpustelnentwicklung erfolgte 12 Tage nach der Infektion.

Verbindungen aus den Tabellen 1 und 2 zeigten gegen Puccinia-Pilze eine gute Wirkung. Unbehandelte aber infizierte Kontrollpflanzen zeigten einen Puccinia-Befall von 100%. Unter anderen hemmten die Verbindungen Nr. 16, 1.24, 1.26, 1.38, 1.75 und 1.144 den Puccinia-Befall auf 0 bis 5%.

Beispiel B2:
Wirkung gegen Cercospora arachidicola auf Erdnusspflanzen
a) Residual-protektive Wirkung

10–15 cm hohe Erdnusspflanzen wurden mit einer aus Spritzpulver der Wirksubstanz hergestellten Spritzbrühe (0,006% Aktivsubstanz) besprüht und 48 Stunden später mit einer Konidiensuspension des Pilzes infiziert. Die infizierten Pflanzen wurden während 72 Stunden bei ca. 21 °C und hoher Luftfeuchtigkeit inkubiert und anschliessend bis zum Auftreten der typischen Blattflecken in einem Gewächshaus aufgestellt. Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100%), zeigten Erdnusspflanzen, die mit Wirkstoffen aus den Tabellen 1 und 2 behandelt wurden, einen stark reduzierten Cercospora-Befall. So verhinderten die Verbindungen Nr. 23, 1.11, 1.15, 1.17, 1.24, 1.26, 1.40, 1.75 und 1.144 in obigen Versuchen das Auftreten von Flecken fast vollständig (8%). Nr. 1.24 zeigte diese Wirkung auch noch bei einer Verdünnung auf 0,002%.

Beispiel B3:
Wirkung gegen Erysiphe graminis auf Gerste
a) Residual-protektive Wirkung

Ca. 8 cm hohe Gerstenpflanzen wurden mit einer aus Spritzpulver des Wirkstoffes hergestellten Spritzbrühe (0,02% Aktivsubstanz) besprüht. Nach 3–4 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22 °C aufgestellt und der Pilzbefall nach 10 Tagen beurteilt. Verbindungen der Formel I zeigten gute Wirkung gegen Erysiphe-Pilze. Unbehandelte, aber infizierte Kontrollpflanzen zeigten einen Erysiphe-Befall von 100%. Verbindungen aus den Tabellen 1 und 2 zeigten eine starke Hemmung des Pilzbefalls, so reduzierten die Verbindungen Nr. 2, 16, 1.15, 1.21, 1.24, 1.26, 1.36 und 2.2 den Befall fast vollständig (0–5%). Am aktivsten erwiesen sich dabei die Verbindungen Nr. 1.24 und 1.36.

Beispiel B4:
Residual-protektive Wirkung gegen Venturia inaequalis auf Apfeltrieben

Apfelstecklinge mit 10–20 cm langen Frischtrieben wurden mit einer aus Spritzpulver des Wirkstoffes hergestellten Spritzbrühe (0,06% Aktivsubstanz) besprüht. Nach 24 Stunden wurden die behandelten Pflanzen mit einer Konidiensuspension des Pilzes infiziert. Die Pflanzen wurden dann während 5 Tagen bei 90–100% relativer Luftfeuchtigkeit inkubiert und während 10 weiteren Tagen in einem Gewächshaus bei 20–24 °C aufgestellt. Der Schorfbefall wurde 15 Tage nach der Infektion beurteilt. Verbindungen Nr. 1, 4, 6, 7, 10, 24, 1.6, 1.8, 1.14, 1.17, 1.21, 1.24, 1.26, 1.28, 1.34, 1.36, 1.40, 1.52, 1.144, 2.3 und andere hemmten den Krankheitsbefall auf weniger als 10%. Unbehandelte aber infizierte Triebe zeigten einen 100%igen Venturia-Befall vollständig. Bei einer Verdünnung auf 0,006% zeigten die Verbindungen Nr. 1.24, 1.26, 1.40 und 2.3 noch volle Wirkung (0% Befall).

Beispiel B5:
Wirkung gegen Botrytis cinerea auf Bohnen
Residual protektive Wirkung

Ca. 10 cm hohe Bohnen-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95–100% relativer Luftfeuchtigkeit und 21 °C erfolgte die Beurteilung des Pilzbefalls. Die Verbindungen aus den Tabellen 1 bis 9 hemmten in vielen Fällen die Pilzinfektion sehr stark. Bei einer Konzentration von 0,02% erwiesen sich z.B. die Verbindungen Nr. 1, 4, 6, 7, 10, 24, 1.1 bis 1.8, 1.14, 1.15, 1.17, 1.19, 1.20, 1.34 und 2.1 als voll wirksam (Krankheitsbefall 0 bis 5%). Befall unbehandelter aber infizierter Bohnenpflanzen betrug 100%. Gleich gut wirken auch die Zwischenprodukte Nr. 16 und 24.

Beispiel B6:
Wirkung gegen Botrytis cinerea an Apfelfrüchten

Künstlich verletzte Äpfel wurden behandelt, indem eine aus Spritzpulver der Wirksubstanz hergestellte Spritzbrühe auf die Verletzungsstellen aufgetropft wurde. Die behandelten Früchte wurden anschliessend mit einer Sporensuspension von Botrytis cinerea inokuliert und während einer

Woche bei einer hohen Luftfeuchtigkeit und ca. 20 °C inkubiert.

Bei der Auswertung wurden die angefaulten Verletzungsstellen gezählt und daraus die fungizide Wirkung der Testsubstanz abgeleitet. Unter anderen hemmten die Verbindungen Nr. 1, 4, 6, 7, 10, 24, 1.1 bis 1.8, 1.14 bis 1.24, 1.26 bis 1.34, 1.36 bis 1.43, 1.51, 1.52, 1.67, 1.68, 1.71, 1.72, 1.75, 1.98, 1.113, 1.123, 1.129, 1.136 bis 1.139, 1.144 bis 1.147, 1.150, 2.1, 2.2, 2.3, 2.9, 2.16, 2.17 und 2.21 bis 2.24 den Pilzbefall im Vergleich zu unbehandelten Kontrollfrüchten (100% Befall) fast vollständig. Gleich gut wirken auch die Zwischenprodukte Nr. 16 und 24.

Beispiel B7:
Wirkung gegen Alternaria solani auf Tomate

Tomatenpflanzen wurden nach dreiwöchiger Anzucht mit einer aus Spritzpulver des Wirkstoffs hergestellten Spritzbrühe (0,06% Aktivsubstanz) besprüht. Nach 24 Stunden wurden die Tomatenpflanzen mit einer Konidiensuspension des Pilzes infiziert. Die Beurteilung der fungiziden Wirkung erfolgte aufgrund des Pilzbefalls nach einer Inkubation der infizierten Pflanzen während 8 Tagen bei hoher Luftfeuchtigkeit und einer Temperatur von 18–22°. Verbindungen aus den Tabellen 1 und 2 bewirkten eine starke Reduktion des Alternariabefalls, so hemmten die Substanzen Nr. 1.23, 1.24 und 1.26 den Befall vollständig (0–5%).

Beispiel B8:
Wirkung gegen Piricularia oryzae auf Reis

Reispflanzen wurden nach zweiwöchiger Anzucht mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach 5 Tagen Inkubation bei 95–100% relativer Luftfeuchtigkeit und 24 °C wurde der Pilzbefall beurteilt. Verbindungen der Formel I zeigten eine gute Hemmung des Piriculariabefalls, so z.B. die Verbindungen Nr. 1.23, 1.24, 1.26, 175 und 1.144; sie reduzierten den Befall auf weniger als 10%.

Beispiel B9:
Körnerschutz: (Grainpreservative Test)
a) Kurzzeittest gegen Schimmelpilze an Feucht-Mais

Trockene, als Tierfutter vorgesehene Maiskörner (Portionen à 80 g) werden in verschliessbaren Plastikbechern mit Wirkstoffen aus allen Tabellen in Form einer wässrigen Suspension, Emulsion oder Lösung gut durchmischt. Die Substanzapplikation wird so bemessen, dass eine Konzentration von 0,06% AS bezüglich des Maistrockengewichts erreicht wird. Ein befeuchteter Papierlappen sorgt für eine feuchtigkeitsgesättigte Atmosphäre in den mit Mais gefüllten und anschliessend verschlossenen Bechern. Nach 2–3 Wochen Inkubation bei ca. 20 °C entwickelt sich bei den nur mit Wasser behandelten Mais-Proben spontan eine Mischpopulation von Schimmelpilzen. Eine künstliche Infektion erübrigt sich. Das Ausmass der Pilzentwicklung nach 3 Wochen dient zur Bewertung der Wirksamkeit der Verbindungen der Formeln I und II'.

b) Langzeittest gegen Schimmelpilze an Feucht-Mais

I. Die Mais-Proben, die nach 3 Wochen keinen Pilzbefall aufweisen, werden während zwei weiteren Monaten inkubiert. Nach jedem Monat erfolgt eine visuelle Bonitur nach denselben Kriterien wie im a-Test.

II. Die Testdurchführung erfolgt grundsätzlich gleich wie unter a und b, doch wird der zu prüfende Wirkstoff der Formeln I oder II' bei 2000, 600 und 200 ppm AS (bezogen auf das Maistrockengewicht) während 6 Monaten geprüft.

Durch Behandlung mit Verbindungen der Formel I aus allen Tabellen wurde die Bildung von Schimmelpilzen an Feuchtmais in allen drei Tests a, bI und bII sowohl kurzfristig (3 Wochen) als auch langfristig (6 Monate) vollständig unterdrückt.

So verhinderten die Verbindungen Nr. 16, 24, 1.14 und 1.15 in allen drei Tests a, bI und bII bei einer Prüfkonzentration von 600 ppm AS den Schimmelpilzbefall fast vollständig (0–5% Befall).

In ähnlichen Versuchen, bei denen anstatt Futtermais wahlweise Futtergetreide (Hafer), Heu, Rübenschnitzel oder Saubohnen verwendet wurden, beobachtete man gleichartige Resultate eines mehrmonatigen Dauerschutzes bei Behandlung mit den obengenannten Wirksubstanzen der Formeln I und II'.

Beispiel B10:
Wirkung gegen Tilletia caries

Tilletiasporen werden in einer Spritzbrühe enthaltend 600 ppm Wirkstoff während 15 min suspendiert. Das Sporen-Substanz-Gemisch wird tropfenweise auf die Oberfläche von fein gesiebter, angefeuchteter Erde in Petrischalen pipettiert. Die so zubereiteten Erdschalen werden bei hoher Luftfeuchtigkeit (95–100%) und einer Temperatur von 20 °C aufgestellt. Nach ca. 10 Tagen wird die Sporenkeimung unter der Lupe beurteilt. Die Wirkung der Testsubstanzen wird auf Grund der Anzahl und der Länge der Keimschläuche ermittelt. Verbindungen der Formel I und II' zeigten eine sehr gute Wirkung gegen Tilletia caries Pilze. So verhinderten in obigem Test unter anderen die Verbindungen Nr. 1, 4, 6, 7, 10, 24, 1.6, 1.8, 1.14, 1.15, 1.17, 1.21, 1.24, 1.26, 1.28, 1.36, 1.40, 1.52 und 2.3 die Ausbildung von Keimschläuchen vollständig.

**Patentansprüche für die Vertragsstaaten: BE, CH, DE, FR, GB, IT, LI, NL**

1. Verbindungen der allgemeinen Formel I,

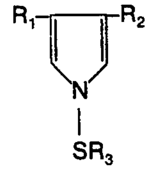

(I)

worin $R_1$ für eines der Fragmente [X,Y,Z(Phenyl)], [X,Y,Z(Biphenyl)], [U,V,W(Pyridyl)], [U,V,W(Furyl)] oder [U,V,W(Thienyl)] steht, wobei X, Y und Z unabhängig voneinander für Wasserstoff, Halogen, $C_1$–$C_4$-Alkyl, $C_1$–$C_3$-Haloalkyl, Di($C_1$–$C_4$-alkyl)amino, Nitro, Cyano, –COO($C_1$–$C_4$-alkyl), –CONC$C_1$–$C_4$-alkyl$)_2$ oder die Gruppe –E–$R_4$ bedeuten, wobei E für –O–, –S–, –SO– oder –$SO_2$– steht, $R_4$ unsubstituiertes oder durch $C_1$–$C_4$-Alkoxy substituiertes $C_1$–$C_6$-Alkyl, unsubstituiertes $C_3$–$C_5$-Alkenyl, unsubstituiertes oder durch Halogen oder Hydroxy substituiertes $C_3$–$C_5$-Alkinyl, [X,Y,Z(Phenyl)] oder –$CH_2$– [X,Y,Z-(Phenyl)] bedeutet; U, V, W unabhängig voneinander für Wasserstoff, Halogen oder $C_1$–$C_4$-Alkyl stehen; $R_2$ für –COO($C_1$–$C_6$-alkyl), –CO–($C_1$–$C_6$-alkyl), –CO–N($C_1$–$C_6$-alkyl$)_2$, Cyano, Nitro, –$SO_2$–($C_1$–$C_6$-alkyl), –P(O)–($C_1$–$C_6$-alkoxy$)_2$ oder für –$SO_2$–N($C_1$–$C_6$-alkyl$)_2$ steht; und $R_3$ $C_1$–$C_3$-Haloalkyl bedeutet.

2. Verbindungen der Formel I nach Anspruch 1, dadurch gekennzeichnet, dass $R_1$ für eines der Fragmente [X,Y,Z(Phenyl)], [X,Y,Z(Biphenyl-4-yl)], [X,Y,Z(Biphenyl-3-yl)], [X,Y,Z(Biphenyl-2-yl)], [U,V,W(2-Pyridyl)], [U,V,W(3-Pyridyl)], [U,V,W(4-Pyridyl)], [U,V,W(2-Furyl)], [U,V,W(3-Furyl)], [U,V,W(2-Thienyl)] oder [U,V,W3-Thienyl] steht, wobei X, Y, Z unabhängig voneinander für Wasserstoff, $C_1$–$C_3$-Alkyl, $C_1$–$C_3$-Haloalkyl, Di($C_1$–$C_3$-alkyl)amino, Nitro, Cyano, –COO($C_1$–$C_3$-alkyl), –CON($C_1$–$C_3$-alkyl$)_2$, $C_1$–$C_4$-Alkoxy, $C_1$–$C_4$-Thioalkyl, $C_3$–$C_5$-Alkenyloxy, $C_3$–$C_5$-Haloalkenyloxy, Phenoxy oder Benzyloxy stehen; U, V, W unabhängig voneinander für Wasserstoff, Halogen oder $C_1$–$C_4$-Alkyl stehen; $R_2$ –COO($C_1$–$C_3$-alkyl), –CO–($C_1$–$C_3$-alkyl), –CO–N($C_1$–$C_3$-alkyl$)_2$ Cyano, Nitro, –$SO_2$–($C_1$–$C_3$-alkyl), –P(O)($C_1$–$C_3$-alkoxy$)_2$ oder –$SO_2$–N($C_1$–$C_3$-alkyl$)_2$ bedeutet; und $R_3$ für $C_1$–$C_3$-Haloalkyl steht.

3. Verbindungen der Formel I nach Anspruch 1, dadurch gekennzeichnet, dass $R_1$ für eines der Fragmente [X,Y,Z(Phenyl)], [X,Y,Z(Biphenyl-4-yl)], [U,V,W(2-Pyridyl)], [U,V,W(3-Pyridyl)], [U,V,W(2-Furyl)] oder [U,V,W(2-Thienyl)] steht; wobei X, Y, Z unabhängig voneinander für Wasserstoff, Fluor, Chlor, Brom, Methyl, Ethyl, Trifluormethyl, –N$(CH_3)_2$, –N$(C_2H_5)_2$, –$COOCH_3$, –$COOC_2H_5$, –CON$(CH_3)_2$, $C_1$–$C_4$-Alkoxy, $C_1$–$C_4$-Alkylthio, $C_3$–$C_5$-Alkenyloxy, $C_3$–$C_5$-Haloalkenyloxy, Phenoxy oder Benzyloxy stehen; U, V, W unabhängig voneinander für Wasserstoff, Chlor, Brom oder $C_1$–$C_2$-Alkyl stehen; $R_2$ –$COOCH_3$, –$COOC_2H_5$, –$COCH_3$, –$COC_2H_5$, –CON$(CH_3)_2$, –CON$(C_2H_5)_2$, Cyano, Nitro, –$SO_2$–$CH_3$, –$SO_2$–$C_2H_5$, –P(O)$(OCH_3)_2$, –P(O)$(OC_2H_5)_2$, –SO–$CH_3$, –$SO_2$–N$(CH_3)_2$ oder –$SO_2$–N$(C_2H_5)_2$ bedeutet; und $R_3$ für $C_1$–$C_2$-Haloalkyl steht.

4. Verbindungen der Formel I nach Anspruch 3, dadurch gekennzeichnet, dass $R_1$ für eines der Molekülfragmente [X,Y,Z(Phenyl)], [U,V,W(2-Pyridyl)], [U,V,W(3-Pyridyl)], [U,V,W(2-Furyl)] oder [U,V,W(2-Thienyl)] steht; wobei X, Y, Z unabhängig voneinander für Fluor, Chlor, Brom, Methyl, Ethyl, Trifluormethyl, $C_1$–$C_3$-Alkoxy oder $C_1$–$C_3$-Alkylthio stehen; U, V, W unabhängig voneinander für Wasserstoff, Chlor, Brom, Methyl oder Ethyl stehen; $R_2$ –$COOCH_3$, $COCH_3$, $NO_2$ oder CN bedeutet; und $R_3$ für $CFCl_2$ oder $CCl_3$ steht.

5. Verbindungen der Formel I nach Anspruch 2, dadurch gekennzeichnet, dass $R_1$ für eines der Fragmente [X,Y,Z(Phenyl)] oder [U,V,W(2-Furyl)] steht; wobei X, Y, Z unabhängig voneinander für Wasserstoff, Chlor, Brom, Methyl, Ethyl, Trifluormethyl, –N$(CH_3)_2$, –$COOCH_3$, –CON$(CH_3)_2$, $C_1$–$C_4$-Alkoxy, $C_1$–$C_4$-Alkylthio, $C_3$-Alkenyloxy, Phenoxy oder Benzyloxy stehen; U, V, W unabhängig voneinander Wasserstoff, Chlor, Brom oder $C_1$–$C_2$-Alkyl bedeuten; $R_2$ für –$COOCH_3$, –$COCH_3$, –CON$(CH_3)_2$, Cyano, Nitro, –$SO_2$–$CH_3$, –P(O)$(OC_2H_5)_2$ oder –$SO_2$–N$(CH_3)_2$ steht; und $R_3$ $CCl_3$, $CCl_2F$, $CCl_2H$, $CClH_2$, $CF_3$, $CF_2H$, $C_2Cl_5$ oder $CCl_2CHCl_2$ bedeutet.

6. Verbindungen der Formel I, nach Anspruch 5, dadurch gekennzeichnet, dass $R_1$ für das Molekülfragment [X,Y,Z(Phenyl)] steht; wobei X, Y, Z unabhängig voneinander für Wasserstoff, Chlor, Brom, Methyl, $C_1$–$C_4$-Alkoxy, $C_1$–$C_4$-Alkylthio oder $C_3$-Alkenyloxy stehen; $R_2$ –$COCH_3$ oder Cyano bedeutet; und $R_3$ für $CCl_3$, $CCl_2F$ oder $CCl_2CHCl_2$ steht.

7. Eine Verbindung der Formel I, nach Anspruch 3, ausgewählt aus der Reihe:
N-Fluordichlormethylsulfenyl-3-(2-allyloxyphenyl)-4-cyanopyrrol,
N-Fluordichlormethylsulfenyl-3-(2-chlorphenyl)-4-cyanopyrrol,
N-Fluordichlormethylsulfenyl-3-phenyl-4-acetylpyrrol,
N-Fluordichlormethylsulfenyl-3-(2-chlorphenyl)-4-acetylpyrrol,
N-Fluordichlormethylsulfenyl-3-(4-methylphenyl)-4-acetylpyrrol,
N-Fluordichlormethylsulfenyl-3-(3-chlorphenyl)-4-acetylpyrrol,
N-Fluordichlormethylsulfenyl-3-(3-methylphenyl)-4-acetylpyrrol,
N-Fluordichlormethylsulfenyl-3-(3-chlorphenyl)-4-methoxycarbonylpyrrol,
N-Fluordichlormethylsulfenyl-3-(2-chlorphenyl)-4-methoxycarbonylpyrrol,
N-Fluordichlormethylsulfenyl-3-(2-chlorphenyl)-4-nitropyrrol,
N-Fluordichlormethylsulfenyl-3-(2-furyl)-4-cyanopyrrol,
N-Trichlormethyl-3-(2-pyridyl)-4-cyanopyrrol,
N-Fluordichlormethylsulfenyl-3-(2,3-dichlorphenyl)-4-cyanopyrrol.

8. Verfahren zur Herstellung der in Anspruch 1 definierten Verbindungen der Formel I, dadurch gekennzeichnet, dass man ein freies Pyrrol der Formel II

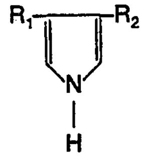

(II)

in Gegenwart einer Base mit einem reaktionsfähigen Säurederivat einer Sulfonsäure der Formel III

$R_3$–S–OH (III)

am Pyrrolstickstoff sulfenyliert, wobei die Substituenten $R_1$, $R_2$ und $R_3$ die unter Formel I angegebenen Bedeutungen haben.

9. Mikrobizides Mittel zur Bekämpfung oder Verhütung eines Befalls von lebenden Pflanzen und/oder zur Konservierung von verderblichem Lagergut pflanzlicher oder tierischer Herkunft, dadurch gekennzeichnet, dass es als mindestens eine aktive Komponente eine der in Anspruch 1 definierten Verbindungen enthält.

10. Mittel gemäss Anspruch 15, dadurch gekennzeichnet, dass es als aktive Komponente mindestens eine der in den Ansprüchen 2 bis 7 definierten Verbindungen enthält.

11. Verfahren zur Bekämpfung oder Verhütung eines Befalls von Kulturpflanzen durch phytopathogene Mikroorganismen, dadurch gekennzeichnet, dass man eine Verbindung der Formel I gemäss einem der Ansprüche 1 bis 7 auf die Pflanze, Pflanzenteile oder deren Standort appliziert.

12. Verfahren zur Konservierung bzw. zum Schutz von Vorrats- oder Lagergut pflanzlicher und/oder tierischer Herkunft vor schädlichen Mikroorganismen durch Behandlung des Gutes mit mindestens einer mikrobizid wirksamen Menge einer Verbindung der Formel I gemäss einem der Ansprüche 1 bis 7.

13. Verwendung von Verbindungen der Formel I gemäss Anspruch 1 zur Bekämpfung und/oder zur präventiven Verhütung eines Befalls von Pflanzen durch Botrytis-Pilze.

14. Pyrrol-Derivate der Formel II′

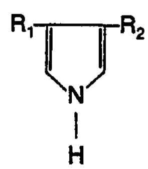

(II′),

worin $R_1$ für eines der Fragmente [X,Y,Z(Phenyl)], [X,Y,Z(Biphenyl)], [U,V,W(Pyridyl)], [U,V,W(Furyl)] oder [U,V,W(Thienyl)] steht, wobei X, Y und Z unabhängig voneinander für Wasserstoff, Halogen, $C_1$–$C_4$-Alkyl, $C_1$–$C_3$-Haloalkyl, Di($C_1$–$C_4$-alkyl)amino, Nitro, Cyano, –COO($C_1$–$C_4$-alkyl), –CON($C_1$–$C_4$-alkyl)$_2$ oder die Gruppe –E–$R_4$ bedeuten, wobei E für –O–, –S–, –SO– oder –$SO_2$– steht, $R_4$ unsubstituiertes oder durch $C_1$–$C_4$-Alkoxy substituiertes $C_1$–$C_6$-Alkyl, unsubstituiertes oder durch Halogen substituiertes $C_3$–$C_5$-Alkenyl, unsubstituiertes oder durch Halogen oder Hydroxy substituiertes $C_3$–$C_5$-Alkinyl; [X,Y,Z(Phenyl)] oder –$CH_2$–[X,Y,Z(Phenyl)] bedeutet, U, V, W unabhängig voneinander für Wasserstoff, Halogen oder $C_1$–$C_4$-Alkyl stehen; $R_2$ für –COO($C_1$–$C_6$-alkyl), –CO–($C_1$–$C_6$-alkyl), –CO–N($C_1$–$C_6$-alkyl)$_2$, Nitro, –$SO_2$–($C_1$–$C_6$-alkyl), –P(O)($C_1$–$C_6$-alkoxy)$_2$, –SO–($C_1$–$C_6$-alkyl) oder für –$SO_2$–N($C_1$–$C_3$-alkyl)$_2$ steht, und in den Fällen, in denen $R_1$ für eines der Fragmente [X,Y,Z(Biphenyl)], [U,V,W(Pyridyl)], [U,V,W(Furyl)] oder [U,V,W(Thienyl)] steht, $R_2$ zusätzlich die Bedeutung Cyano hat.

15. 3-(2-Methylthiophenyl)-4-cyanopyrrol.

16. 3-(2-Methoxyphenyl)-4-cyanopyrrol.

**Patentansprüche (für den Vertragsstaat: Österreich)**

1. Mikrobizides Mittel zur Bekämpfung oder Verhütung eines Befalls von lebenden Pflanzen und/oder zur Konservierung von verderblichem Lagergut pflanzlicher oder tierischer Herkunft, dadurch gekennzeichnet, dass es als mindestens eine aktive Komponente eine der Verbindungen der allgemeinen Formel I,

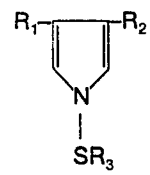

(I)

enthält,
worin $R_1$ für eines der Fragmente [X,Y,Z(Phenyl)], [X,Y,Z(Biphenyl)], [U,V,W(Pyridyl)], [U,V,W(Furyl)] oder [U,V,W(Thienyl)] steht, wobei X, Y und Z unabhängig voneinander für Wasserstoff, Halogen, $C_1$–$C_4$-Alkyl, $C_1$–$C_3$-Haloalkyl, Di($C_1$–$C_4$-alkyl)amino, Nitro, Cyano, –COO($C_1$–$C_4$-alkyl), –CON($C_1$–$C_4$-alkyl)$_2$ oder die Gruppe –E–$R_4$ bedeuten, wobei E für –O–, –S–, –SO– oder –$SO_2$– steht, $R_4$ unsubstituiertes $C_1$–$C_6$-Alkyl, unsubstituiertes oder durch Halogen substituiertes $C_3$–$C_5$-Alkenyl, unsubstituiertes oder durch Halogen oder Hydroxy substituiertes $C_3$–$C_5$-Alkinyl, [X,Y,Z(Phenyl)] oder –$CH_2$– [X,Y,Z(Phenyl)] bedeutet; U,V,W unabhängig voneinander für Wasserstoff, Halogen oder $C_1$–$C_4$-Alkyl stehen; $R_2$ für –COO($C_1$–$C_6$-alkyl), –CO–($C_1$–$C_6$-alkyl), –CO–N($C_1$–$C_6$-alkyl)$_2$, Cyano, Nitro, –$SO_2$–($C_1$–$C_6$-alkyl), –P(O)–($C_1$–$C_6$-alkoxy)$_2$ oder für –$SO_2$–N($C_1$–$C_6$-alkyl)$_2$ steht; und $R_3$ $C_1$–$C_3$-Haloalkyl bedeutet.

2. Mittel nach Anspruch 1, enthaltend mindestens eine der Verbindungen der Formel I, worin $R_1$ für eines der Fragmente [X,Y,Z(Phenyl)], [X,Y,Z(Biphenyl-4-yl)], [X,Y,Z(Biphenyl-3-yl)], [X,Y,Z(Biphenyl-2-yl)], [U,V,W(2-Pyridyl)], [U,V,W(3-Pyridyl)], [U,V,W(4-Pyridyl)], [U,V,W(2-Furyl)], [U,V,W(3-Furyl)], [U,V,W(2-Thienyl)] oder [U,V,W3-Thienyl] steht, wobei X, Y, Z unabhängig voneinander für Wasserstoff, $C_1$–$C_3$-Alkyl, $C_1$–$C_3$-Haloalkyl, Di($C_1$–$C_3$-alkyl)amino, Nitro, Cyano, –COO($C_1$–$C_3$-alkyl, –CON($C_1$–$C_3$-alkyl)$_2$, $C_1$–$C_4$-Alkoxy, $C_1$–$C_4$-

Thioalkyl, $C_3$–$C_5$-Alkenyloxy, $C_3$–$C_5$-Haloalkenyloxy, Phenoxy oder Benzyloxy stehen; U, V, W unabhängig voneinander für Wasserstoff, Halogen oder $C_1$–$C_4$-Alkyl stehen; $R_2$ –COO($C_1$–$C_3$-alkyl), –CO–($C_1$–$C_3$-alkyl), –CO–N($C_1$–$C_3$-alkyl)$_2$ Cyano, Nitro, –$SO_2$–($C_1$–$C_3$-alkyl), –P(O)($C_1$–$C_3$-alkoxy)$_2$ oder –$SO_2$–N($C_1$–$C_3$-alkyl)$_2$ bedeutet; und $R_3$ für $C_1$–$C_3$-Haloalkyl steht.

3. Mittel nach Anspruch 1, enthaltend mindestens eine der Verbindungen der Formel I, worin $R_1$ für eines der Fragmente [X,Y,Z(Phenyl)], [X,Y,Z(Biphenyl-4-yl)], [U,V,W(2-Pyridyl)], [U,V,W(3-Pyridyl)], [U,V,W(2-Furyl)] oder [U,V,W(2-Thienyl)] steht; wobei X, Y, Z unabhängig voneinander für Wasserstoff, Fluor, Chlor, Brom, Methyl, Ethyl, Trifluormethyl, –N($CH_3$)$_2$, –N($C_2H_5$)$_2$, –$COOCH_3$, –$COOC_2H_5$, –CON($CH_3$)$_2$, $C_1$–$C_4$-Alkoxy, $C_1$–$C_4$-Alkylthio, $C_3$–$C_5$-Alkenyloxy, $C_3$–$C_5$-Haloalkenyloxy, Phenoxy oder Benzyloxy stehen; U, V, W unabhängig voneinander für Wasserstoff, Chlor, Brom oder $C_1$–$C_2$-Alkyl stehen; $R_2$ –$COOCH_3$, –$COOC_2H_5$, –$COCH_3$, –$COC_2H_5$, –CON($CH_3$)$_2$, –CON($C_2H_5$)$_2$, Cyano, Nitro, –$SO_2$–$CH_3$, –$SO_2$–$C_2H_5$, –P(O)($OCH_3$)$_2$, –P(O)($OC_2H_5$)$_2$, –SO–$CH_3$, –$SO_2$–N($CH_3$)$_2$ oder –$SO_2$–N($C_2H_5$)$_2$ bedeutet; und $R_3$ für $C_1$–$C_2$-Haloalkyl steht.

4. Mittel nach Anspruch 3, enthaltend eine der Verbindungen der Formel I, worin $R_1$ für eines der Molekülfragmente [X,Y,Z(Phenyl)], [U,V,W(2-Pyridyl)], [U,V,W(3-Pyridyl)], [U,V,W(2-Furyl)] oder [U,V,W(2-Thienyl)] steht; wobei X, Y, Z unabhängig voneinander für Fluor, Chlor, Brom, Methyl, Ethyl, Trifluormethyl, $C_1$–$C_3$-Alkoxy oder $C_1$–$C_3$-Alkylthio stehen; U, V, W unabhängig voneinander für Wasserstoff, Chlor, Brom, Methyl oder Ethyl stehen; $R_2$ –$COOCH_3$, $NO_2$, $COCH_3$ oder CN bedeutet; und $R_3$ für $CFCl_2$ oder $CCl_3$ steht.

5. Mittel nach Anspruch 2, enthaltend mindestens eine der Verbindungen der Formel I, worin $R_1$ für eines der Fragmente [X,Y,Z(Phenyl)] oder [U,V,W(2-Furyl)] steht; wobei X, Y, Z unabhängig voneinander für Wasserstoff, Chlor, Brom, Methyl, Ethyl, Trifluormethyl, –N($CH_3$)$_2$, –$COOCH_3$, –CON($CH_3$)$_2$, $C_1$–$C_4$-Alkoxy, $C_1$–$C_4$-Alkylthio, $C_3$-Alkenyloxy, Phenoxy oder Benzyloxy stehen; U, V, W unabhängig voneinander Wasserstoff, Chlor, Brom oder $C_1$–$C_2$-Alkyl bedeuten; $R_2$ für –$COOCH_3$, –$COCH_3$, –CON($CH_3$)$_2$, Cyano, Nitro, –$SO_2$–$CH_3$, –P(O)($OC_2H_5$)$_2$ oder –$SO_2$–N($CH_3$)$_2$ steht; und $R_3$ $CCl_3$, $CCl_2F$, $CCl_2H$, $CClH_2$, $CF_3$, $CF_2H$, $C_2Cl_5$ oder $CCl_2CHCl_2$ bedeutet.

6. Mittel nach Anspruch 4, enthaltend mindestens eine der Verbindungen der Formel I, worin $R_1$ für das Molekülfragment [X,Y,Z(Phenyl)] steht; wobei X, Y, Z unabhängig voneinander für Wasserstoff, Chlor, Brom, Methyl, $C_1$–$C_4$-Alkoxy, $C_1$–$C_4$-Alkylthio oder $C_3$-Alkenyloxy stehen; $R_2$ –$COCH_3$ oder Cyano bedeutet; und $R_3$ für $CCl_3$, $CCl_2F$ oder $CCl_2CHCl_2$ steht.

7. Mittel nach Anspruch 1, dadurch gekennzeichnet, dass es als mindestens eine aktive Komponente eine Verbindung der Formel I enthält, ausgewählt aus der Reihe:
N-Fluordichlormethylsulfenyl-3-(2-allyloxyphenyl)-4-cyanopyrrol,
N-Fluordichlormethylsulfenyl-3-(2-chlorphenyl)-4-cyanopyrrol,
N-Fluordichlormethylsulfenyl-3-phenyl-4-acetylpyrrol,
N-Fluordichlormethylsulfenyl-3-(2-chlorphenyl)-4-acetylpyrrol,
N-Fluordichlormethylsulfenyl-3-(4-methylphenyl)-4-acetylpyrrol,
N-Fluordichlormethylsulfenyl-3-(3-chlorphenyl)-4-acetylpyrrol,
N-Fluordichlormethylsulfenyl-3-(3-methylphenyl)-4-acetylpyrrol,
N-Fluordichlormethylsulfenyl-3-(3-chlorphenyl)-4-methoxycarbonylpyrrol,
N-Fluordichlormethylsulfenyl-3-(2-chlorphenyl)-4-methoxycarbonylpyrrol,
N-Fluordichlormethylsulfenyl-3-(2-chlorphenyl)-4-nitropyrrol,
N-Fluordichlormethylsulfenyl-3-(2-furyl)-4-cyanopyrrol,
N-Trichlormethyl-3-(2-pyridyl)-4-cyanopyrrol,
N-Fluordichlormethylsulfenyl-3-(2,3-dichlorphenyl)-4-cyanopyrrol.

8. Verfahren zur Herstellung der in Anspruch 1 definierten Verbindungen der Formel I, dadurch gekennzeichnet, dass man ein freies Pyrrol der Formel II

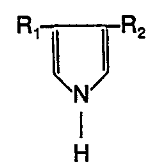

(II)

in Gegenwart einer Base mit einem reaktionsfähigen Säurederivat einer Sulfensäure der Formel III

$$R_3\text{–S–OH} \qquad \text{(III)}$$

am Pyrrolstickstoff sulfenyliert, wobei die Substituenten $R_1$, $R_2$ und $R_3$ die unter Formel I angegebenen Bedeutungen haben.

9. Verfahren nach Anspruch 8, dadurch gekennzeichnet, dass man als reaktionsfähiges Sulfensäurederivat einen Niederalkylsäureester oder ein Säurehalogenid der Säure III einsetzt.

10. Verfahren nach Anspruch 9, dadurch gekennzeichnet, dass man das Säurechlorid der Säure III einsetzt.

11. Verfahren nach einem der Ansprüche 8 bis 10, dadurch gekennzeichnet, dass man als Base ein Alkali- oder Erdalkalicarbonat oder ein tertiäres Amin einsetzt.

12. Verfahren nach einem der Ansprüche 8 bis 11, dadurch gekennzeichnet, dass man die Reaktion in Gegenwart eines reaktionsinerten Lösungsmittels durchführt.

13. Verfahren nach einem der Ansprüche 8 bis 12, dadurch gekennzeichnet, dass man als Reaktionskatalysator 4-Dimethylaminopyridin zusetzt.

14. Verfahren nach einem der Ansprüche 8 bis 13, dadurch gekennzeichnet, dass man die Umsetzung bei Temperaturen von $-30°$ bis $+100\,°C$ durchführt.

15. Verfahren nach Anspruch 14, dadurch gekennzeichnet, dass man die Reaktion bei Temperaturen von $-10°$ bis $+20\,°C$ durchführt.

16. Verfahren zur Bekämpfung oder Verhütung eines Befalls von Kulturpflanzen durch phytopathogene Mikroorganismen, dadurch gekennzeichnet, dass man eine Verbindung der Formel I gemäss einem der Ansprüche 1 bis 6 auf die Pflanze, Pflanzenteile oder deren Standort appliziert.

17. Verfahren zur Konservierung bzw. zum Schutz von Vorrats- oder Lagergut pflanzlicher und/oder tierischer Herkunft vor schädlichen Mikroorganismen durch Behandlung des Gutes mit mindestens einer mikrobizid wirksamen Menge einer der in den Ansprüchen 1 bis 6 definierten Verbindung der Formel I.

18. Verfahren nach Anspruch 17, dadurch gekennzeichnet, dass es sich bei dem Vorrats- oder Lagergut um Fleisch- oder Fischverarbeitungsprodukte handelt.

19. Verfahren nach Anspruch 17, dadurch gekennzeichnet, dass es sich bei dem Vorrats- oder Lagergut um geerntete Pflanzen oder um Pflanzenteile handelt, deren natürlicher Lebenszyklus unterbrochen wurde.

20. Verwendung von Verbindungen der Formel I gemäss Anspruch 1 zur Bekämpfung und/oder zur präventiven Verhütung eines Befalls von Mikroorganismen.

21. Verwendung nach Anspruch 20 von Verbindungen der Formel I, die in einem der Ansprüche 2 bis 7 definiert sind.

22. Verwendung gemäss Anspruch 20, dadurch gekennzeichnet, dass es sich bei den Mikroorganismen vom Botrytis-Pilze handelt.

23. Mikrobizides Mittel zur Bekämpfung oder Verhütung eines Befalls von lebenden Pflanzen und/oder zur Konservierung von verderblichem Lagergut pflanzlicher oder tierischer Herkunft, dadurch gekennzeichnet, dass es als mindestens eine aktive Komponente eines der Pyrrol-Derivate der Formel II′

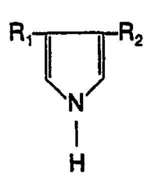

(II′),

enthält,
worin $R_1$ für eines der Fragmente
[X,Y,Z(Phenyl)], [X,Y,Z(Biphenyl)],
[U,V,W(Pyridyl)], [U,V,W(Furyl)] oder
[U,V,W(Thienyl)]
steht, wobei X, Y und Z unabhängig voneinander für Wasserstoff, Halogen, $C_1$–$C_4$-Alkyl, $C_1$–$C_3$-Haloalkyl, Di($C_1$–$C_4$-alkyl)amino, Nitro, Cyano, –COO($C_1$–$C_4$-alkyl), –CON($C_1$–$C_4$-alkyl)$_2$ oder die Gruppe –E–$R_4$ bedeuten, wobei E für –O–, –S–, –SO– oder –$SO_2$– steht, $R_4$ unsubstituiertes oder durch $C_1$–$C_4$-Alkoxy substituiertes $C_1$–$C_6$-Alkyl, unsubstituiertes oder durch Halogen substituiertes $C_3$–$C_5$-Alkenyl, unsubstituiertes oder durch Halogen oder Hydroxy substituiertes $C_3$–$C_5$-Alkinyl; [X,Y,Z(Phenyl)] oder –$CH_2$–[X,Y,Z(Phenyl)] bedeutet, U,V,W unabhängig voneinander für Wasserstoff, Halogen oder $C_1$–$C_4$-Alkyl stehen; $R_2$ für
–COO($C_1$–$C_6$-alkyl), –CO–($C_1$–$C_6$-alkyl),
–CO–N($C_1$–$C_6$-alkyl)$_2$, Nitro, –$SO_2$–($C_1$–$C_6$-alkyl),
–P(O)($C_1$–$C_6$-alkoxy)$_2$, –SO–($C_1$–$C_6$-alkyl) oder für –$SO_2$–N($C_1$–$C_3$-alkyl)$_2$ steht, und in den Fällen, in denen $R_1$ für eines der Fragmente
[X,Y,Z(Biphenyl)], [U,V,W(Pyridyl)],
[U,V,W(Furyl)] oder [U,V,W(Thienyl)]
steht, $R_2$ zusätzlich die Bedeutung Cyano hat.

24. Mittel enthaltend als Pyrrolderivat der Formel II′ mindestens eine der Verbindungen:
3-(2-Methylthiophenyl)-4-cyanopyrrol oder
3-(2-Methoxyphenyl)-4-cyanopyrrol.

**Claims for Contracting States BE, CH, DE, FR, GB, IT, LI, NL**

1. Compounds of the general formula I

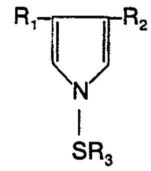

(I)

wherein $R_1$ is a fragment
[X,Y,Z(phenyl)], [X,Y,Z(biphenyl)],
[U,V,W(pyridyl)], [U,V,W(furyl)] or
[U,V,W(thienyl)],
wherein X, Y and Z are each independently hydrogen, halogen, $C_1$–$C_4$alkyl, $C_1$–$C_3$haloalkyl, di($C_1$–$C_4$alkyl)amino, nitro, cyano, –COO($C_1$–$C_4$alkyl), –CON($C_1$–$C_4$alkyl)$_2$ or the group –E–$R_4$, wherein E is –O–, –S–, –SO– or –$SO_2$–, $R_4$ is $C_1$–$C_6$alkyl which is unsubstituted or substituted by $C_1$–$C_4$alkoxy, unsubstituted $C_3$–$C_5$alkenyl, $C_3$–$C_5$alkynyl which is unsubstituted or substituted by halogen or hydroxy, or is [X,Y,Z(phenyl)] or –$CH_2$–[X,Y,Z-(phenyl)]; U, V and W are each independently hydrogen, halogen or $C_1$–$C_4$alkyl; $R_2$ is
–COO($C_1$–$C_6$alkyl), –CO–($C_1$–$C_6$alkyl),
–CO–N($C_1$–$C_6$alkyl)$_2$, cyano, nitro,
–$SO_2$–($C_1$–$C_6$alkyl), –P(O)–($C_1$–$C_6$alkoxy)$_2$
or –$SO_2$–N($C_1$–$C_6$alkyl)$_2$; and $R_3$ is $C_1$–$C_3$haloalkyl.

2. Compounds of the formula I according to claim 1, wherein $R_1$ is a fragment
[X,Y,Z(phenyl)], [X,Y,Z(biphenyl-4-yl)],
[X,Y,Z(biphenyl-3-yl)], [X,Y,Z(biphenyl-2-yl)],
[U,V,W(2-pyridyl)], [U,V,W(3-pyridyl)],
[U,V,W(4-pyridyl)], [U,V,W(2-furyl)],
[U,V,W(3-furyl)], [U,V,W(2-thienyl)] or
[U,V,W(3-thienyl)],
wherein X, Y and Z are each independently
hydrogen, $C_1$–$C_3$alkyl, $C_1$–$C_3$haloalkyl,
di($C_1$–$C_3$alkyl)amino, nitro, cyano,
–COO($C_1$–$C_3$alkyl), –CON($C_1$–$C_3$alkyl)$_2$,
$C_1$–$C_4$alkoxy, $C_1$–$C_4$alkylthio, $C_3$–$C_5$alkenyloxy,
$C_3$–$C_5$haloalkenyloxy, phenoxy or benzyloxy;
U, V and W are each independently
hydrogen, halogen or $C_1$–$C_4$alkyl;
$R_2$ is –COO($C_1$–$C_3$alkyl), –CO–($C_1$–$C_3$alkyl),
–CO–N($C_1$–$C_3$alkyl)$_2$, cyano, nitro,

$-SO_2-(C_1-C_3alkyl)$, $-P(O)(C_1-C_3alkoxy)_2$ or $-SO_2-N(C_1-C_3alkyl)_2$; and $R_3$ is $C_1-C_3$haloalkyl.

3. Compounds of the formula I according to claim 1, wherein $R_1$ is a fragment [X,Y,Z(phenyl)], [X,Y,Z(biphenyl-4-yl)], [U,V,W(2-pyridyl)], [U,V,W(3-pyridyl)], [U,V,W(2-furyl)] or [U,V,W(2-thienyl)], wherein X, Y and Z are each independently hydrogen, fluorine, chlorine, bromine, methyl, ethyl, trifluoromethyl, $-N(CH_3)_2$, $-N(C_2H_5)_2$, $-COOCH_3$, $-COOC_2H_5$, $-CON(CH_3)_2$, $C_1-C_4$alkoxy, $C_1-C_4$alkylthio, $C_3-C_5$alkenyloxy, $C_3-C_5$haloalkenyloxy, phenoxy or benzyloxy; U, V and W are each independently hydrogen, chlorine, bromine or $C_1-C_2$alkyl; $R_2$ is $-COOCH_3$, $-COOC_2H_5$, $-COCH_3$, $-COC_2H_5$, $-CON(CH_3)_2$, $-CON(C_2H_5)_2$, cyano, nitro, $-SO_2-CH_3$, $-SO_2-C_2H_5$, $-P(O)(OCH_3)_2$, $-P(O)(OC_2H_5)_2$, $-SO-CH_3$, $-SO_2-N(CH_3)_2$ or $-SO_2-N(C_2H_5)_2$; and $R_3$ is $C_1-C_2$haloalkyl.

4. Compounds of the formula I according to claim 3, wherein $R_1$ is a molecule fragment [X,Y,Z(phenyl)], [U,V,W(2-pyridyl)], [U,V,W(3-pyridyl)], [U,V,W(2-furyl)] or [U,V,W(2-thienyl)], wherein X, Y and Z are each independently fluorine, chlorine, bromine, methyl, ethyl, trifluoromethyl, $C_1-C_3$alkoxy or $C_1-C_3$alkylthio; U, V and W are each independently hydrogen, chlorine, bromine, methyl or ethyl; $R_2$ is $-COOCH_3$, $COCH_3$, $NO_2$ or CN; and $R_3$ is $CFCl_2$ or $CCl_3$.

5. Compounds of the formula I according to claim 2, wherein $R_1$ is a fragment [X,Y,Z(phenyl)] or [U,V,W(2-furyl)], wherein X, Y and Z are each independently hydrogen, chlorine, bromine, methyl, ethyl, trifluoromethyl, $-N(CH_3)_2$, $-COOCH_3$, $-CON(CH_3)_2$, $C_1-C_4$-alkoxy, $C_1-C_4$alkylthio, $C_3$alkenyloxy, phenoxy or benzyloxy; U, V and W are each independently hydrogen, chlorine, bromine or $C_1-C_2$alkyl; $R_2$ is $-COOCH_3$, $-COCH_3$, $-CON(CH_3)_2$, cyano, nitro, $-SO_2-CH_3$, $P(O)(OC_2H_5)_2$ or $-SO_2-N(CH_3)_2$; and $R_3$ is $CCl_3$, $CCl_2F$, $CCl_2H$, $CClH_2$, $CF_3$, $CF_2H$, $C_2Cl_5$ or $CCl_2CHCl_2$.

6. Compounds of the formula I according to claim 5, wherein $R_1$ is the molecule fragment [X,Y,Z(phenyl)], wherein X, Y and Z are each independently hydrogen, chlorine, bromine, methyl, $C_1-C_4$alkoxy, $C_1-C_4$alkylthio or $C_3$alkenyloxy; $R_2$ is $-COOH_3$ or cyano; and $R_3$ is $CCl_3$, $CCl_2F$ or $CCl_2CHCl_2$.

7. A compound of the formula I according to claim 3, selected from the group consisting of:
N-fluorodichloromethylsulfenyl-3-(2-allyloxyphenyl)-4-cyanopyrrole,
N-fluorodichloromethylsulfenyl-3-(2-chlorophenyl)-4-cyanopyrrole,
N-fluorodichloromethylsulfenyl-3-phenyl-4-acetylpyrrole,
N-fluorodichloromethylsulfenyl-3-(2-chlorophenyl)-4-acetylpyrrole,
N-fluorodichloromethylsulfenyl-3-(4-methylphenyl)-4-acetylpyrrole,
N-fluorodichloromethylsulfenyl-3-(3-chlorophenyl)-4-acetylpyrrole,
N-fluorodichloromethylsulfenyl-3-(3-methylphenyl)-4-acetylpyrrole,
N-fluorodichloromethylsulfenyl-3-(3-chlorophenyl)-4-methoxycarbonylpyrrole,
N-fluorodichloromethylsulfenyl-3-(2-chlorophenyl)-4-methoxycarbonylpyrrole,
N-fluorodichloromethylsulfenyl-3-(2-chlorophenyl)-4-nitropyrrole,
N-fluorodichloromethylsulfenyl-3-(2-furyl)-4-cyanopyrrole,
N-trichloromethyl-3-(2-pyridyl)-4-cyanopyrrole,
N-fluorodichloromethylsulfenyl-3-(2,3-dichlorophenyl)-4-cyanopyrrole.

8. A process for the preparation of the compounds of the formula I defined in claim 1, which process comprises sulfenylating a free pyrrole of the formula II

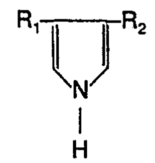

(II)

wherein $R_1$ and $R_2$ are as defined for formula I, at the pyrrole nitrogen with a reactive acid derivative of a sulfenic acid of the formula III

$$R_3-S-OH \quad (III),$$

wherein $R_3$ is as defined for formula I, in the presence of a base.

9. A microbicidal composition for controlling or preventing an attack on living plants and/or for preserving perishable storable goods of vegetable or animal origin, which composition contains, as at least one active component, one of the compounds defined in claim 1.

10. A composition according to claim 15, which contains as active component at least one of the compounds defined in any one of claims 2 to 7.

11. A method of controlling or preventing an attack on cultivated plants by phytopathogenic microorganisms, which method comprises applying to the plants, to parts of the plants or to the locus thereof a compound of the formula I according to any one of claims 1 to 7.

12. A method of preserving or protecting storable goods of vegetable and/or animal origin from attack by harmful microorganisms, which method comprises treating said goods with a least a microbicidally effective amount of a compound of the formula I according to any one of claims 1 to 7.

13. The use of compounds of the formula I according to claim 1 for controlling and/or preventing an attack on plants by Botrytis fungi.

14. Pyrrole derivatives of the formula II′

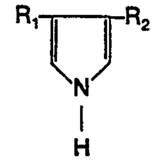

(II′),

wherein $R_1$ is a fragment [X,Y,Z(phenyl)], [X,Y,Z(biphenyl)], [U,V,W(pyridyl)], [U,V,W(furyl)] or [U,V,W(thienyl)], wherein X, Y and Z are each independently hydrogen, halogen, $C_1$–$C_4$alkyl, $C_1$–$C_3$haloalkyl, di($C_1$–$C_4$alkyl)amino, nitro, cyano, –COO($C_1$–$C_4$alkyl), –CON($C_1$–$C_4$alkyl)$_2$ or the group –E–$R_4$, wherein E is –O–, –S–, –SO– or –$SO_2$–, $R_4$ is $C_1$–$C_6$alkyl which is unsubstituted or substituted by $C_1$–$C_4$alkoxy, $C_3$–$C_5$alkenyl which is unsubstituted or substituted by halogen, $C_3$–$C_5$alkynyl which is unsubstituted or substituted by halogen or hydroxy, or is [X,Y,Z-(phenyl)] or –$CH_2$–[X,Y,Z(phenyl)], and U, V and W are each independently hydrogen, halogen or $C_1$–$C_4$alkyl; $R_2$ is –COO($C_1$–$C_6$alkyl), –CO–($C_1$–$C_6$alkyl), –CO–N($C_1$–$C_6$alkyl)$_2$, nitro, –$SO_2$–($C_1$–$C_6$alkyl), –P(O)($C_1$–$C_6$alkoxy)$_2$, –SO–($C_1$–$C_6$alkyl) or –$SO_2$–N($C_1$–$C_3$alkyl)$_2$, and, where $R_1$ is a fragment [X,Y,Z(biphenyl)], [U,V,W(pyridyl)], [U,V,W(furyl)] or [U,V,W(thienyl)], $R_2$ may also be cyano.

15. 3-(2-Methylthiophenyl)-4-cyanopyrrole.

16. 3-(2-Methoxyphenyl)-4-cyanopyrrole.

**Claims for Contracting State AT**

1. A microbicidal composition for controlling or preventing an attack on living plants and/or for preserving perishable storable goods of vegetable or animal origin, which composition contains, as at least one active component, one of the compounds of the general formula I

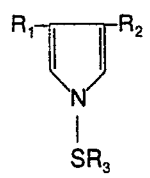

(I)

wherein $R_1$ is a fragment [X,Y,Z(phenyl)], [X,Y,Z(biphenyl)], [U,V,W(pyridyl)], [U,V,W(furyl)] or [U,V,W(thienyl)], wherein X, Y and Z are each independently hydrogen, halogen, $C_1$–$C_4$alkyl, $C_1$–$C_3$haloalkyl, di($C_1$–$C_4$alkyl)amino, nitro, cyano, –COO($C_1$–$C_4$alkyl), –CON($C_1$–$C_4$alkyl)$_2$ or the group –E–$R_4$, wherein E is –O–, –S–, –SO– or –$SO_2$–, $R_4$ is unsubstituted $C_1$–$C_6$alkyl, $C_3$–$C_5$alkenyl which is unsubstituted or substituted by halogen, $C_3$–$C_5$alkynyl which is unsubstituted or substituted by halogen or hydroxy, or is [X,Y,Z(phenyl)] or –$CH_2$–[X,Y,Z(phenyl)]; U, V and W are each independently hydrogen, halogen or $C_1$–$C_4$alkyl; $R_2$ is –COO($C_1$–$C_6$alkyl), –CO–($C_1$–$C_6$alkyl), –CO–N($C_1$–$C_6$alkyl)$_2$, cyano, nitro, –$SO_2$–($C_1$–$C_6$alkyl), –P(O)–($C_1$–$C_6$alkoxy)$_2$ or –$SO_2$–N($C_1$–$C_6$alkyl)$_2$; and $R_3$ is $C_1$–$C_3$haloalkyl.

2. A composition according to claim 1, which contains at least one of the compounds of the formula I wherein $R_1$ is a fragment [X,Y,Z(phenyl)], [X,Y,Z(biphenyl-4-yl)], [X,Y,Z(biphenyl-3-yl)], [X,Y,Z(biphenyl-2-yl)], [U,V,W(2-pyridyl)], [U,V,W(3-pyridyl)], [U,V,W(4-pyridyl)], [U,V,W(2-furyl)], [U,V,W(3-furyl)], [U,V,W(2-thienyl)] or [U,V,W(3-thienyl)], wherein X, Y and Z are each independently hydrogen, $C_1$–$C_3$alkyl, $C_1$–$C_3$haloalkyl, di($C_1$–$C_3$alkyl)amino, nitro, cyano, –COO($C_1$–$C_3$alkyl), –CON($C_1$–$C_3$alkyl)$_2$, $C_1$–$C_4$alkoxy, $C_1$–$C_4$alkylthio, $C_3$–$C_5$alkenyloxy, $C_3$–$C_5$haloalkenyloxy, phenoxy or benzyloxy; U, V and W are each independently hydrogen, halogen or $C_1$–$C_4$alkyl; $R_2$ is –CO($C_1$–$C_3$alkyl), –CO–($C_1$–$C_3$alkyl), –CO–N($C_1$–$C_3$alkyl)$_2$, cyano, nitro, –$SO_2$–($C_1$–$C_3$alkyl), –P(O)($C_1$–$C_3$alkoxy)$_2$ or –$SO_2$–N($C_1$–$C_3$alkyl)$_2$; and $R_3$ is $C_1$–$C_3$haloalkyl.

3. A composition according to claim 1, which contains at least one of the compounds of the formula I wherein $R_1$ is a fragment [X,Y,Z(phenyl)], [X,Y,Z(biphenyl-4-yl)], [U,V,W(2-pyridyl)], [U,V,W(3-pyridyl)], [U,V,W(2-furyl)] or [U,V,W(2-thienyl)], wherein X, Y and Z are each independently hydrogen, fluorine, chlorine, bromine, methyl, ethyl, trifluoromethyl, –N($CH_3$)$_2$, –N($C_2H_5$)$_2$, –$COOCH_3$, –$COOC_2H_5$, –CON($CH_3$)$_2$, $C_1$–$C_4$alkoxy, $C_1$–$C_4$alkylthio, $C_3$–$C_5$alkenyloxy, $C_3$–$C_5$haloalkenyloxy, phenoxy or benzyloxy; U, V and W are each independently hydrogen, chlorine, bromine or $C_1$–$C_2$alkyl; $R_2$ is –$COOCH_3$, –$COOC_2H_5$, –$COCH_3$, –$COC_2H_5$, –CON($CH_3$)$_2$, –CON($C_2H_5$)$_2$, cyano, nitro, –$SO_2$–$CH_3$, –$SO_2$–$C_2H_5$, –P(O)($OCH_3$)$_2$, –P(O)($OC_2H_5$)$_2$, –SO–$CH_3$, –$SO_2$–N($CH_3$)$_2$ or –$SO_2$–N($C_2H_5$)$_2$; and $R_3$ is $C_1$–$C_2$haloalkyl.

4. A composition according to claim 3, which contains one of the compounds of the formula I wherein $R_1$ is a molecule fragment [X,Y,Z(phenyl)], [U,V,W(2-pyridyl)], [U,V,W(3-pyridyl)], [U,V,W(2-furyl)] or [U,V,W(2-thienyl)], wherein X, Y and Z are each independently fluorine, chlorine, bromine, methyl, ethyl, trifluoromethyl, $C_1$–$C_3$alkoxy or $C_1$–$C_3$alkylthio; U, V and W are each independently hydrogen, chlorine, bromine, methyl or ethyl; $R_2$ is –$COOCH_3$, $NO_2$, $COCH_3$ or CN; and $R_3$ is $CFCl_2$ or $CCl_3$.

5. A composition according to claim 2, which contains at least one of the compounds of the formula I wherein $R_1$ is a fragment [X,Y,Z(phenyl)] or [U,V,W(2-furyl)], wherein X, Y and Z are each independently hydrogen, chlorine, bromine, methyl, ethyl, trifluoromethyl, –N($CH_3$)$_2$, –$COOCH_3$, –CON($CH_3$)$_2$, $C_1$–$C_4$alkoxy, $C_1$–$C_4$alkylthio, $C_3$alkenyloxy, phenoxy or benzyloxy; U, V and W are each independently hydrogen, chlorine, bromine or $C_1$–$C_2$alkyl; $R_2$ is –$COOCH_3$, –$COCH_3$, –CON($CH_3$)$_2$, cyano, nitro, –$SO_2$–$CH_3$, P(O)($OC_2H_5$)$_2$ or –$SO_2$–N($CH_3$)$_2$; and $R_3$ is $CCl_3$, $CCl_2F$, $CCl_2H$, $CClH_2$, $CF_3$, $CF_2H$, $C_2Cl_5$ or $CCl_2CHCl_2$.

6. A composition according to claim 4, which contains at least one of the compounds of the formula I wherein $R_1$ is the molecule fragment [X,Y,Z(phenyl)], wherein X, Y and Z are each independently hydrogen, chlorine, bromine, methyl, $C_1$–$C_4$alkoxy, $C_1$–$C_4$alkylthio or $C_3$alkenyloxy; $R_2$ is $-COCH_3$ or cyano; and $R_3$ is $CCl_3$, $CCl_2F$ or $CCl_2CHCl_2$.

7. A composition according to claim 1, which contains, as at least one active component, a compound of the formula I selected from the group consisting of:
N-fluorodichloromethylsulfenyl-3-(2-allyloxyphenyl)-4-cyanopyrrole,
N-fluorodichloromethylsulfenyl-3-(2-chlorophenyl)-4-cyanopyrrole,
N-fluorodichloromethylsulfenyl-3-phenyl-4-acetylpyrrole,
N-fluorodichloromethylsulfenyl-3-(2-chlorophenyl)-4-acetylpyrrole,
N-fluorodichloromethylsulfenyl-3-(4-methylphenyl)-4-acetylpyrrole,
N-fluorodichloromethylsulfenyl-3-(3-chlorophenyl)-4-acetylpyrrole,
N-fluorodichloromethylsulfenyl-3-(3-methylphenyl)-4-acetylpyrrole,
N-fluorodichloromethylsulfenyl-3-(3-chlorophenyl)-4-methoxycarbonylpyrrole,
N-fluorodichloromethylsulfenyl-3-(2-chlorophenyl)-4-methoxycarbonylpyrrole,
N-fluorodichloromethylsulfenyl-3-(2-chlorophenyl)-4-nitropyrrole,
N-fluorodichloromethylsulfenyl-3-(2-furyl)-4-cyanopyrrole,
N-trichloromethyl-3-(2-pyridyl)-4-cyanopyrrole,
N-fluorodichloromethylsulfenyl-3-(2,3-dichlorophenyl)-4-cyanopyrrole.

8. A process for the preparation of the compounds of the formula I defined in claim 1, which process comprises sulfenylating a free pyrrole of the formula II

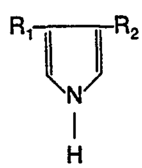

(II)

wherein $R_1$ and $R_2$ are as defined for formula I, at the pyrrole nitrogen with a reactive acid derivative of a sulfenic acid of the formula III

$$R_3\text{–S–OH} \qquad \text{(III)},$$

wherein $R_3$ is as defined for formula I, in the presence of a base.

9. A process according to claim 8, wherein a lower alkyl ester or a halide of the acid III is used as the reactive sulfenic acid derivative.

10. A process according to claim 9, wherein the chloride of the acid III is used.

11. A process according to any one of claims 8 to 10, wherein an alkali metal carbonate, an alkaline earth metal carbonate or a tertiary amine is used as the base.

12. A process according to any one of claims 8 to 11, wherein the reaction is carried out in the presence of an inert solvent.

13. A process according to any one of claims 8 to 12, wherein 4-dimethylaminopyridine is added as a reaction catalyst.

14. A process according to any one of claims 8 to 13, wherein the reaction is carried out at temperatures of from $-30°$ to $+100\,°C$.

15. A process according to claim 14, wherein the reaction is carried out at temperatures of from $-10°$ to $+20\,°C$.

16. A method of controlling or preventing an attack on cultivated plants by phytopathogenic microorganisms, which method comprises applying to the plants, to parts of the plants or to the locus thereof a compound of the formula I according to any one of claims 1 to 6.

17. A method of preserving or protecting storable goods of vegetable and/or animal origin from attack by harmful microorganisms, which method comprises treating said goods with at least a microbicidally effective amount of one of the compounds of the formula I defined in any one of claims 1 to 6.

18. A method according to claim 17, wherein the storable goods are processed meat or fish products.

19. A method according to claim 17, wherein the storable goods are harvested plants or parts of plants whose natural life cycle has been interrupted.

20. The use of compounds of the formula I according to claim 1 for controlling and/or preventing an attack by microorganisms.

21. The use according to claim 20 of compounds of the formula I defined in any one of claims 2 to 7.

22. The use according to claim 20, wherein the microorganisms are Botrytis fungi.

23. A microbicidal composition for controlling or preventing an attack on living plants and/or for preserving perishable storable goods of vegetable or animal origin, which composition contains, as at least one active component, one of the pyrrole derivatives of the formula II′

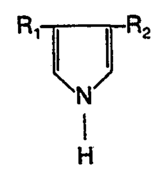

(II′),

wherein $R_1$ is a fragment
[X,Y,Z(phenyl)], [X,Y,Z(biphenyl)], [U,V,W(pyridyl)], [U,V,W(furyl)] or [U,V,W(thienyl)],
wherein X, Y and Z are each independently hydrogen, halogen, $C_1$–$C_4$alkyl, $C_1$–$C_3$haloalkyl, di($C_1$–$C_4$alkyl)amino, nitro, cyano, $-COO(C_1$–$C_4$alkyl), $-CON(C_1$–$C_4$alkyl$)_2$ or the group $-E-R_4$, wherein E is –O–, –S–, –SO– or $-SO_2-$, $R_4$ is $C_1$–$C_6$alkyl which is unsubstituted or substituted by $C_1$–$C_4$alkoxy, $C_3$–$C_5$alkenyl which is unsubstituted or substituted by halogen, $C_3$–$C_5$alkynyl which is unsubstituted or substituted by halogen or hydroxy, or is [X,Y,Z–(phenyl)] or $-CH_2-$[X,Y,Z(phenyl)] and U, V and W are each independently hydrogen, halogen or

$C_1$–$C_4$alkyl; $R_2$ is –COO($C_1$–$C_6$alkyl), –CO–($C_1$–$C_6$alkyl), –CO–N($C_1$–$C_6$alkyl)$_2$, nitro, –$SO_2$–($C_1$–$C_6$alkyl), –P(O)($C_1$–$C_6$alkoxy)$_2$, –SO–($C_1$–$C_6$alkyl) or –$SO_2$–N($C_1$–$C_3$alkyl)$_2$, and, where $R_1$ is a fragment [X,Y,Z(biphenyl)], [U,V,W(pyridyl)], [U,V,W(furyl)] or [U,V,W(thienyl)], $R_2$ may also be cyano.

24. A composition that contains as the pyrrole derivative of the formula II′ at least one of the compounds:
3-(2-methylthiophenyl)-4-cyanopyrrole and
3-(2-methoxyphenyl)-4-cyanopyrrole.

**Revendications pour les Etats Contractants BE, CH, DE, FR, GB, IT, LI, NL**

1. Composés de formule générale

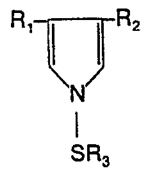

(I)

dans laquelle $R_1$ représente l'un des fragments
[X,Y,Z(phényle)], [X,Y,Z(biphényle)],
[U,V,W(pyridyle)], [U,V,W(furyle)] ou
[U,V,W(thiényle)]
dans lesquels X, Y et Z représentent chacun, indépendamment les uns des autres, l'hydrogène, un halogène, un groupe alkyle en C 1–C 4, halogénoalkyle en C1–C 3, di-(alkyle en C 1–C 4)-amino, nitro, cyano, –COO(alkyle en C 1–C 4), –CON(alkyle en C 1–C 4)$_2$ ou le groupe –E–$R_4$ dans lequel E représente –O–, –S–, –SO– ou –$SO_2$, $R_4$ représente un groupe alkyle en C 1–C 6 non substitué ou substitué par un groupe alcoxy en C 1–C 4, un groupe alcényle en C 3–C 5 non substitué ou un groupe alcynyle en C 3–C 5 non substitué ou substitué par des halogènes ou des groupes hydroxy, un groupe [X,Y,Z(phényle)] ou –$CH_2$–[X,Y,Z(phényle)]; U, V et W représentent chacun, indépendamment les uns des autres, l'hydrogène, un halogène ou un groupe alkyle en C 1–C 4; $R_2$ représente un groupe –COO(alkyle en C 1–C 6), –CO-(alkyle en C 1–C 6), –CO–N(alkyle en C 1–C 6)$_2$, cyano, nitro, –$SO_2$–(alkyle en C 1–C 6), –P(O)–(alcoxy en C 1–C 6)$_2$ ou –$SO_2$–N(alkyle en C 1–C 6)$_2$; et $R_3$ représente un groupe halogénoalkyle en C 1–C 3.

2. Composés de formule I selon la revendication 1, caractérisés en ce que $R_1$ représente l'un des fragments
[X,Y,Z(phényle)], [X,Y,Z(biphényle-4-yle)],
[X,Y,Z(biphényle-3-yle)],
[X,Y,Z(biphényle-2-yle)], [U,V,W(2-pyridyle)],
[U,V,W(3-pyridyle)], [U,V,W(4-pyridyle)],
[U,V,W(2-furyle)], [U,V,W(3-furyle)],
[U,V,W(2-thiényle)], ou [U,V,W(3-thiényle)]
dans lesquels X, Y et Z représentent chacun, indépendamment les uns des autres, l'hydrogène, un groupe alkyle en C 1–C 3, halogénoalkyle en C 1–C 3, di-(alkyle en C 1–C 3)-amino, nitro, cyano, –COO(alkyle en C 1–C 3), –CON(alkyle en C 1–C 3)$_2$, alcoxy en C 1–C 4, thioalkyle en C 1–C 4, alcényloxy en C 3–C 5, halogénoalcényloxy en C 3–C 5, phénoxy ou benzyloxy; U, V et W représentent chacun, indépendamment les uns des autres, l'hydrogène, un halogène ou un groupe alkyle en C 1–C 4; $R_2$ représente un groupe –COO(alkyle en C 1–C 3), –CO–(alkyle en C 1–C 3), –CON(alkyle en C 1–C 3)$_2$, cyano, nitro, –$SO_2$–(alkyle en C 1–C 3), –P(O)(alcoxy en C 1–C 3)$_2$ ou –$SO_2$–N(alkyle en C 1–C 3)$_2$; et $R_3$ représente un groupe halogénoalkyle en C 1–C 3.

3. Composés de formule I selon la revendication 1, caractérisés en ce que $R_1$ représente l'un des fragments
[X,Y,Z(phényle)], [X,Y,Z(biphényle-4-yle)],
[U,V,W(2-pyridyle)], [U,V,W(3-pyridyle)],
[U,V,W(2-furyle)] ou [U,V,W(2-thiényle)];
X, Y et Z représentent chacun, indépendamment les uns des autres, l'hydrogène, le fluor, le chlore, le brome, un groupe méthyle, éthyle, trifluorométhyle, –N($CH_3$)$_2$, –N($C_2H_5$)$_2$, –$COOCH_3$, –$COC_2H_5$, –CON($CH_3$)$_2$, alcoxy en C 1–C 4, alkylthio en C 1–C 4, alcényloxy en C 3–C 5, halogénoalcényloxy en C 3–C 5, phénoxy ou benzyloxy; U, V, W représentent chacun, indépendamment les uns des autres, l'hydrogène, le chlore, le brome ou un groupe alkyle en C 1–C 2; $R_2$ représente un groupe –$COOCH_3$, –$COOC_2H_5$, –$COCH_3$, –$COC_2H_5$, –CON($CH_3$)$_2$, –CON($C_2H_5$)$_2$, cyano, nitro, –$SO_2$–$CH_3$, –$SO_2$–$C_2H_5$, –P(O)($OCH_3$)$_2$, –P(O)($OC_2H_5$)$_2$, –SO–$CH_3$, –$SO_2$–N($CH_3$)$_2$ ou –$SO_2$–N($C_2H_5$)$_2$; et $R_3$ représente un groupe halogénoalkyle en C 1–C 2.

4. Composés de formule I selon la revendication 3, caractérisés en ce que $R_1$ représente l'un des fragments moléculaires
[X,Y,Z(phényle)], [U,V,W(2-pyridyle)],
[U,V,W(3-pyridyle)], [U,V,W(2-furyle)] ou
[U,V,W(2-thiényle)]
dans lesquels X, Y, Z représentent chacun, indépendamment les uns des autres, le fluor, le chlore, le brome, un groupe méthyle, éthyle, trifluorométhyle, alcoxy en C 1–C 3 ou alkylthio en C 1–C 3 et U, V, W représentent chacun, indépendamment les uns des autres, l'hydrogène, le chlore, le brome, un groupe méthyle ou éthyle; $R_2$ représente un groupe –$COOCH_3$, $COCH_3$, $NO_2$ ou CN et $R_3$ représente un groupe $CFCl_2$ ou $CCl_3$.

5. Composés de formule I selon la revendication 2, caractérisés en ce que $R_1$ représente l'un des fragments [X,Y,Z(phényle)] ou [U,V,W(2-furyle)] dans lesquels X, Y, Z représentent chacun, indépendamment les uns des autres, l'hydrogène, le chlore, le brome, un groupe méthyle, éthyle, trifluorométhyle, –N($CH_3$)$_2$, –$COOCH_3$, –CON($CH_3$)$_2$, alcoxy en C 1–C 4, alkylthio en C 1–C 4, alcényloxy en C 3, phénoxy ou benzyloxy et U, V, W représentent chacun, indépendamment les uns des autres, l'hydrogène, le chlore, le brome ou un groupe alkyle en C 1–C 2; $R_2$ représente un groupe –$COOCH_3$, –$COCH_3$, –CON($CH_3$)$_2$, cyano, nitro, –$SO_2$–$CH_3$, –P(O)($OC_2H_5$)$_2$ ou –$SO_2$–N($CH_3$)$_2$; et $R_3$ représente un groupe $CCl_3$, $CCl_2F$, $CCl_2H$, $CClH_2$, $CF_3$, $CF_2H$, $C_2Cl_5$ ou $CCl_2CHCl_2$.

6. Composés de formule I selon la revendication 5, caractérisés en ce que $R_1$ représente le frag-

ment moléculaire [X,Y,Z(phényle)] dans lequel X, Y, Z représentent chacun, indépendamment les uns des autres, l'hydrogène, le chlore, le brome, un groupe méthyle, alcoxy en C 1–C 4, alkylthio en C 1–C 4 ou alcényloxy en C 3; $R_2$ représente un groupe $-COCH_3$ ou cyano et $R_3$ un groupe $CCl_3$, $CCl_2F$ ou $CCl_2CHCl_2$.

7. Un composé de formule I selon la revendication 3, choisi dans le groupe suivant:
N-fluorodichlorométhylsulfényl-3-(2-allyloxyphényl)-4-cyanopyrrole,
N-fluorodichlorométhylsulfényl-3-(2-chlorophényl)-4-cyanopyrrole,
N-fluorodichlorométhylsulfényl-3-phényl-4-acétylpyrrole,
N-fluorodichlorométhylsulfényl-3-(2-chlorophényl)-4-acétylpyrrole,
N-fluorodichlorométhylsulfényl-3-(4-méthylphényl)-4-acétylpyrrole,
N-fluorodichlorométhylsulfényl-3-(3-chlorophényl)-4-acétylpyrrole,
N-fluorodichlorométhylsulfényl-3-(3-méthylphényl)-4-acétylpyrrole,
N-fluorodichlorométhylsulfényl-3-(3-chlorophényl)-4-méthoxycarbonylpyrrole,
N-fluorodichlorométhylsulfényl-3-(2-chlorophényl)-4-méthoxycarbonylpyrrole,
N-fluorodichlorométhylsulfényl-3-(2-chlorophényl)-4-nitropyrrole,
N-fluorodichlorométhylsulfényl-3-(2-furyl)-4-cyanopyrrole,
N-trichlorométhyl-3-(2-pyridyl)-4-cyanopyrrole,
N-fluorodichlorométhylsulfényl-3-(2,3-dichlorophényl)-4-cyanopyrrole.

8. Procédé de préparation des composés de formule I définie dans la revendication 1, caractérisé en ce que l'on sulfényle sur l'azote pyrrolique un pyrrole libre de formule II

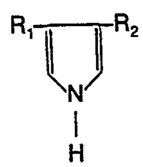

(II)

en présence d'une base, à l'aide d'un dérivé réactif d'un acide sulfénique de formule III

$$R_3\text{–S–OH} \qquad (III)$$

les symboles $R_1$, $R_2$ et $R_3$ ayant les significations indiquées en référence à la formule I.

9. Produit microbicide pour combattre ou prévenir une attaque des végétaux vivants et/ou pour conserver des denrées périssables d'origine végétale ou animale, caractérisé en ce qu'il contient au moins un composant actif consistant en l'un des composés définis dans la revendication 1.

10. Produit selon la revendication 15, caractérisé en ce qu'il contient au moins un composant actif consistant en l'un des composés définis dans les revendications 2 à 7.

11. Procédé pour combattre ou prévenir une attaque de végétaux cultivés par des microorganismes phytopathogènes, caractérisé en ce que l'on applique sur la plante, des parties de la plante ou son habitant un composé de formule I selon l'une des revendications 1 à 7.

12. Procédé pour conserver ou protéger des réserves ou des stocks d'origine végétale ou animale contre les microorganismes nuisibles par traitement de la marchandise à l'aide d'une quantité efficace d'un composé de formule I selon l'une des revendications 1 à 7.

13. Utilisation des composés de formule I selon la revendication 1, pour combattre et/ou prévenir une attaque de végétaux par des mycètes Botrytis.

14. Dérivés du pyrrole répondant à la formule II'

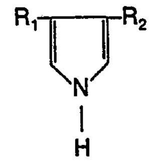

(II'),

dans laquelle $R_1$ représente l'un des fragments [X,Y,Z(phényle)], [X,Y,Z(biphényle)], [U,V,W(pyridyle)], [U,V,W(furyle)] ou [U,V,W(thiényle)]
dans lesquels X, Y et Z représentent chacun, indépendamment les uns des autres, l'hydrogène, un halogène, un groupe alkyle en C 1–C 4, halogénoalkyle en C 1–C 3, di-(alkyle en C 1–C 4)-amino, nitro, cyano, –COO(alkyle en C 1–C 4), $-CON(\text{alkyle en C 1–C 4})_2$ ou le groupe $-E-R_4$ dans lequel E représente –O–, –S–, –SO– ou $-SO_2-$, $R_4$ représente un groupe alkyle en C 1–C 6 non substitué ou substitué par un groupe alcoxy en C 1–C 4, un groupe alcényle en C 3–C 5 non substitué ou substitué par un halogène, un groupe alcynyle en C 3–C 5 non substitué ou substitué par un halogène ou par un groupe hydroxy; un groupe [X,Y,Z(phényle)] ou $-CH_2-$[X,Y,Z(phényle)], U, V, W représentent chacun, indépendamment les uns des autres, l'hydrogène, un halogène ou un groupe alkyle en C 1–C 4; $R_2$ représente un groupe –COO(alkyle en C 1–C 6), –CO–(alkyle en C 1–C 6), $-CO-N(\text{alkyle en C 1–C 6})_2$, nitro, $-SO_2-$(alkyle en C 1–C 6), $-P(O)(\text{alcoxy en C 1–C 6})_2$, –SO–(alkyle en C 1–C 6) ou $-SO_2-N(\text{alkyle en C 1–C 3})_2$ et dans les cas où $R_1$ représente l'un des fragments [X,Y,Z(biphényle)], [U,V,W(pyridyle)], [U,V,W(furyle)] ou [U,V,W(thiényle)], $R_2$ peut en outre représenter un groupe cyano.

15. Le 3-(2-méthylthiophényl)-4-cyanopyrrole.

16. Le 3-(2-méthoxyphényl)-4-cyanopyrrole.

**Revendications pour l'Etat contractant: AT**

1. Produit microbicide pour combattre ou prévenir une infestation de végétaux vivants et/ou pour conserver des denrées périssables d'origine végétale ou animale, caractérisé en ce qu'il contient au moins un composant actif consistant en l'un des composés de formule générale I

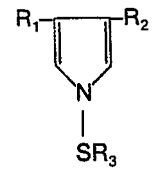

(I)

dans laquelle $R_1$ représente l'un des fragments [X,Y,Z(phényle)], [X,Y,Z(biphényle)], [U,V,W(pyridyle)], [U,V,W(furyle)] ou [U,V,W(thiényle)]
dans lesquels X, Y et Z représentent chacun, indépendamment les uns des autres, l'hydrogène, un halogène, un groupe alkyle en C 1–C 4, halogénoalkyle en C 1–C 3, di-(alkyle en C 1–C 4)-amino, nitro, cyano, –COO(alkyle en C 1–C 4), –CON(alkyle en C 1–C 4)$_2$ ou le groupe E–$R_4$ dans lequel E représente –O–, –S–, –SO– ou –$SO_2$–, $R_4$ représente un groupe alkyle en C 1–C 6 non substitué, un groupe alcényle en C 3–C 5 non substitué ou substitué par un halogène, un groupe alcynyle en C 3–C 5 non substitué ou substitué par un halogène ou un groupe hydroxy, un groupe [X,Y,Z(phényle)] ou –$CH_2$–[X,Y,Z(phényle)]; U, V et W représentent chacun, indépendamment les uns des autres, l'hydrogène, un halogène ou un groupe alkyle en C 1–C 4; $R_2$ représente un groupe –COO(alkyle en C 1–C 6), –CO–(alkyle en C 1–C 6), –CO–N(alkyle en C 1–C 6)$_2$, cyano, nitro, –$SO_2$–(alkyle en C 1–C 6), –P(O)(alcoxy en C 1–C 6)$_2$ ou –$SO_2$–N(alkyle en C 1–C 6)$_2$ et $R_3$ représente un groupe halogénoalkyle en C 1–C 3.

2. Produit selon la revendication 1, caractérisé en ce qu'il contient au moins un des composés de formule I dans laquelle $R_1$ représente l'un des fragments
[X,Y,Z(phényle)], [X,Y,Z(biphényle-4-yle)], [X,Y,Z(biphényle-3-yle)], [X,Y,Z(biphényle-2-yle)], [U,V,W(2-pyridyle)], [U,V,W(3-pyridyle)], [U,V,W(4-pyridyle)], [U,V,W(2-furyle)], [U,V,W(3-furyle)], [U,V,W(2-thiényle)] ou [U,V,W(3-thiényle)]
dans lesquels X, Y et Z représentent chacun, indépendamment les uns des autres, l'hydrogène, un groupe alkyle en C 1–C 3, halogénoalkyle en C 1–C 3, di-(alkyle en C 1–C 3)-amino, nitro, cyano, –COO(alkyle en C 1–C 3), –CON(alkyle en C 1–C 3)$_2$, alcoxy en C 1–C 4, thioalkyle en C 1–C 4, alcényloxy en C 3–C 5, halogénoalcényloxy en C 3–C 5, phénoxy ou benzyloxy; U, V et W représentent chacun, indépendamment les uns des autres, l'hydrogène, un halogène ou un groupe alkyle en C 1–C 4; $R_2$ représente un groupe –COO(alkyle en C 1–C 3), –CO–(alkyle en C 1–C 3), –CON(alkyle en C 1–C 3)$_2$, cyano, nitro, –$SO_2$–(alkyle en C 1–C 3), –P(O)(alcoxy en C 1–C 3)$_2$ ou –$SO_2$–N(alkyle en C 1–C 3)$_2$; et $R_3$ représente un groupe halogénoalkyle en C 1–C 3.

3. Produit selon la revendication 1, contenant au moins un des composés de formule I dans laquelle $R_1$ représente l'un des fragments
[X,Y,Z(phényle)], [X,Y,Z(biphényle-4-yle)], [U,V,W(2-pyridyle)], [U,V,W(3-pyridyle)], [U,V,W(2-furyle)] ou [U,V,W(2-thiényle)];
X, Y et Z représentent chacun, indépendamment les uns des autres, l'hydrogène, le fluor, le chlore, le brome, un groupe méthyle, éthyle, trifluorométhyle, –$N(CH_3)_2$, –$N(C_2H_5)_2$, –$COOCH_3$, –$COC_2H_5$, –$CON(CH_3)_2$, alcoxy en C 1–C 4, alkylthio en C 1–C 4, alcényloxy en C 3–C 5, halogénoalcényloxy en C 3–C 5, phénoxy ou benzyloxy; U, V, W représentent chacun, indépendamment les uns des autres, l'hydrogène, le chlore, le brome ou un groupe alkyle en C 1–C 2; $R_2$ représente un groupe –$COOCH_3$, –$COOC_2H_5$, –$COCH_3$, –$COC_2H_5$, –$CON(CH_3)_2$, –$CON(C_2H_5)_2$, cyano, nitro, –$SO_2$–$CH_3$, –$SO_2$–$C_2H_5$, –$P(O)(OCH_3)_2$, –$P(O)(OC_2H_5)_2$, –SO–$CH_3$, –$SO_2$–$N(CH_3)_2$ ou –$SO_2$–$N(C_2H_5)_2$; et $R_3$ représente un groupe halogénoalkyle en C 1–C 2.

4. Produit selon la revendication 3, contenant l'un des composés de formule I dans laquelle $R_1$ représente l'un des fragments moléculaires [X,Y,Z(phényle)], [U,V,W(2-pyridyle)], [U,V,W(3-pyridyle)], [U,V,W(2-furyle)] ou [U,V,W(2-thiényle)]
dans lesquels X, Y et Z représentent chacun, indépendamment les uns des autres, le fluor, le chlore, le brome, un groupe méthyle, éthyle, trifluorométhyle, alcoxy en C 1–C 3 ou alkylthio en C 1–C 3 et U, V, W représentent chacun, indépendamment les uns des autres, l'hydrogène, le chlore, le brome, un groupe méthyle ou éthyle; $R_2$ représente un groupe –$COOCH_3$, $COCH_3$, $NO_2$ ou CN; et $R_3$ représente un groupe $CFCl_2$ ou $CCl_3$.

5. Produit selon la revendication 2, contenant au moins un des composés de formule I dans laquelle $R_1$ représente l'un des fragments [X,Y,Z(phényle)] ou [U,V,W(2-furyle)] dans lequels X, Y, Z représentent chacun, indépendamment les uns des autres, l'hydrogène le chlore, le brome, un groupe méthyle, éthyle, trifluorométhyle, –$N(CH_3)_2$, –$COOCH_3$, –$CON(CH_3)_2$, alcoxy en C 1–C 4, alkylthio en C 1–C 4, alcényloxy en C 3, phénoxy ou benzyloxy; et U, V, W représentent chacun, indépendamment les uns des autres, l'hydrogène, le chlore, le brome ou un groupe alkyle en C 1–C 2; $R_2$ représente un groupe
–$COOCH_3$, –$COCH_3$, –$CON(CH_3)_2$, cyano, nitro, –$SO_2$–$CH_3$, –$P(O)(OC_2H_5)_2$ ou –$SO_2$–$N(CH_3)_2$;
et $R_3$ représente un groupe $CCl_3$, $CCl_2F$, $CCl_2H$, $CClH_2$, $CF_3$, $CF_2H$, $C_2Cl_5$ ou $CCl_2CHCl_2$.

6. Produit selon la revendication 4, contenant au moins un des composés de formule I dans laquelle $R_1$ représente le fragment moléculaire [X,Y,Z(phényle)] dans lequel X, Y, Z représentent chacun, indépendamment les uns des autres, l'hydrogène, le chlore, le brome, un groupe méthyle, alcoxy en C 1–C 4, alkylthio en C 1–C 4 ou alcényloxy en C 3; $R_2$ représente un groupe –$COCH_3$ ou cyano; et $R_3$ un groupe $CCl_3$, $CCl_2F$ ou $CCl_2CHCl_2$.

7. Produit selon la revendication 1, caractérisé en ce qu'il contient au moins un composant actif consistant en un composé de formule I choisi dans le groupe suivant:
N-fluorodichlorométhylsulfényl-3-(2-allyloxyphényl)-4-cyanopyrrole,
N-fluorodichlorométhylsulfényl-3-(2-chlorophényl)-4-cyanopyrrole,
N-fluorodichlorométhylsulfényl-3-phényl-4-acétylpyrrole,
N-fluorodichlorométhylsulfényl-3-(2-chlorophényl)-4-acétylpyrrole,
N-fluorodichlorométhylsulfényl-3-(4-méthylphényl)-4-acétylpyrrole,
N-fluorodichlorométhylsulfényl-3-(3-chlorophényl)-4-acétylpyrrole,

N-fluorodichlorométhylsulfényl-3-(3-méthylphényl)-4-acétylpyrrole,
N-fluorodichlorométhylsulfényl-3-(3-chlorophényl)-4-méthoxycarbonylpyrrole,
N-fluorodichlorométhylsulfényl-3-(2-chlorophényl)-4-méthoxycarbonylpyrrole,
N-fluorodichlorométhylsulfényl-3-(2-chlorophényl)-4-nitropyrrole,
N-fluorodichlorométhylsulfényl-3-(2-furyl)-4-cyanopyrrole,
N-trichlorométhyl-3-(2-pyridyl)-4-cyanopyrrole,
N-fluorodichlorométhylsulfényl-3-(2,3-dichlorophényl)-4-cyanopyrrole.

8. Procédé de préparation des composés de formule I définie dans la revendication 1, caractérisé en ce que l'on sulfényle sur l'azote pyrrolique un pyrrole libre de formule II

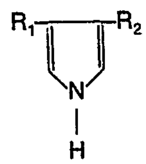

(II)

en présence d'une base, à l'aide d'un dérivé réactif d'un acide sulfénique de formule III

$$R_3\text{–S–OH} \qquad \text{(III)}$$

les symboles $R_1$, $R_2$ et $R_3$ ayant les significations indiquées en référence à la formule I.

9. Procédé selon la revendication 8, caractérisé en ce que l'on utilise en tant que dérivé réactif d'acide sulfénique un ester alkylique inférieur ou un halogénure de l'acide III.

10. Procédé selon la revendication 9, caractérisé en ce que l'on utilise le chlorure de l'acide III.

11. Procédé selon l'une des revendications 8 à 10, caractérisé en ce que l'on utilise en tant que base un carbonate alcalin ou alcalino-terreux ou une amine tertiaire.

12. Procédé selon l'une des revendications 8 à 11, caractérisé en ce que l'on effectue la réaction en présence d'un solvant inerte dans la réaction.

13. Procédé selon l'une des revendications 8 à 12, caractérisé en ce que l'on utilise en tant que catalyseur de réaction la 4-diméthylaminopyridine.

14. Procédé selon l'une des revendications 8 à 13, caractérisé en ce que l'on effectue la réaction à des températures de −30 à +100 °C.

15. Procédé selon la revendication 14, caractérisé en ce que l'on effectue la réaction à des températures de −10 à +20 °C.

16. Procédé pour combattre ou prévenir une attaque de végétaux cultivés par des microorganismes phytopathogènes, caractérisé en ce que l'on applique sur la plante, des parties de la plante ou son habitat un composé de formule I selon l'une des revendications 1 à 6.

17. Procédé pour conserver ou protéger des réserves ou des stocks d'origine végétale ou animale contre les microorganismes nuisibles par traitement de la marchandise à l'aide d'une quantité efficace d'un composé de formule I selon l'une des revendications 1 à 6.

18. Procédé selon la revendication 17, caractérisé en ce que les réserves ou stocks consistent en produits de transformation de viandes ou de poissons.

19. Procédé selon la revendication 17, caractérisé en ce que les stocks ou réserves consistent en végétaux ou parties de végétaux récoltés et dont le cycle de vie naturel a été interrompu.

20. Utilisation des composés de formule I selon la revendication 1 pour combattre et/ou prévenir une attaque par des microorganismes.

21. Utilisation selon la revendication 20, de composés de formule I définis dans l'une des revendications 2 à 7.

22. Utilisation selon la revendication 20, caractérisée en ce que les microorganismes sont des mycètes Botrytis.

23. Produit microbicide pour combattre ou prévenir une attaque de végétaux vivants et/ou pour conserver des denrées périssables d'origine végétale ou animale, caractérisé en ce qu'il contient au moins un composant actif consistant en l'un des dérivés du pyrrole de formule II'

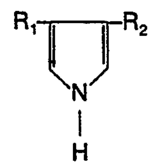

(II'),

dans laquelle $R_1$ représente l'un des fragments [X,Y,Z(phényle)], [X,Y,Z(biphényle)], [U,V,W(pyridyle)], [U,V,W(furyle)] ou [U,V,W(thiényle)]
dans lesquels X, V et Z représentent chacun, indépendamment les uns des autres, l'hydrogène, un halogène, un groupe alkyle en C 1–C 4, halogénoalkyle en C 1–C 3, di-(alkyle en C 1–C 4)-amino, nitro, cyano, –COO(alkyle en C 1–C 4), –CON(alkyle en C 1–C 4)$_2$ ou le groupe –E–$R_4$ dans lequel E représente –O–, –S–, –SO– ou –$SO_2$–, $R_4$ représente un groupe alkyle en C 1–C 6 non substitué ou substitué par un groupe alcoxy en C 1–C 4, un groupe alcényle en C 3–C 5 non substitué ou substitué par un halogène, un groupe alcynyle en C 3–C 5 non substitué ou substitué par un halogène ou par un groupe hydroxy; un groupe [X,Y,Z(phényle)] ou –$CH_2$–[X,Y,Z(phényle)], U, V, W représentent chacun, indépendamment les uns des autres, l'hydrogène, un halogène ou un groupe alkyle en C 1–C 4; $R_2$ représente un groupe –COO–alkyle en C 1–C 6), –CO–(alkyle en C 1–C 6), –CO–N(alkyle en C 1–C 6)$_2$, nitro, –$SO_2$–(alkyle en C 1–C 6), –P(O)(alkoxy en C 1–C 6)$_2$, –SO–(alkyle en C 1–C 6) ou –$SO_2$–N(alkyle en C 1–C 3)$_2$ et dans les cas où $R_1$ représente l'un des fragments [X,Y,Z(biphényle)], [U,V,W(pyridyle)], [U,V,W(furyle)] ou [U,V,W(thiényle)], $R_2$ peut en outre représenter un groupe cyano.

24. Produit contenant en tant que dérivé du pyrrole de formule II' au moins l'un des composés:
3-(2-méthylthiophényl)-4-cyanopyrrole, ou
3-(2-méthoxyphényl)-4-cyanopyrrole.